# EUROPEAN PATENT SPECIFICATION

(11) **EP 4 204 070 B1**
(45) Date of publication and mention of the grant of the patent: **06.08.2025**
(21) Application number: 21762759.5
(22) Date of filing: 17.08.2021
(51) Int. Cl.: A61N 1/36, A61N 1/04, A61B 5/256, A61B 5/296, A61B 5/395, A61B 5/00, A61H 23/02

(54) **MUSCLE STIMULATION**
MUSKELSTIMULATION
STIMULATION DE MUSCLES

(30) Priority: 28.08.2020 GB 202013574; 23.06.2021 GB 202108994
(43) Date of publication of application: 05.07.2023
(73) Proprietor: Knitregen Limited, Kirkham Preston Lancashire PR4 2EB (GB)
(72) Inventor: SALISBURY, Laura, Preston Lancashire PR4 2EB (GB)
(74) Representative: Barker Brettell LLP
(86) International application number: PCT/GB2021/052125
(87) International publication number: WO 2022/043658

(56) References cited:
- WO-A2-2019/134033
- US-A1- 2014 142 652
- US-A1- 2014 277 220
- US-A1- 2017 136 265

## Description

### Technical field

The present invention relates to wearable articles configured to provide stimulation to a muscle of a wearer. The present invention relates particularly, but not exclusively, to garments configured to provide muscle stimulation to a wearer suffering from muscle weakness.

### Background

Limb weakness, such as upper limb weakness, is a debilitating consequence of stroke and other neurological disorders. 87% of stroke survivors are affected by such muscle weakness, with over 40% of survivors left with persisting weakness. Left untreated, weakened muscles can stiffen further, making it more difficult to move, more painful for the survivor, and resulting in increased disability over time. The depression of strength, control and reflexes can lead to hyperreflexia/increased tone in some cases, leading to clear changes in limb posture and positioning that can be both painful and debilitating.

Stimulating the affected muscles of a person suffering from limb weakness can improve flexibility, strength and coordination. However, extensive physiotherapy is not available and/or practical for all affected patients. Systems have been proposed that aim to stimulate the sensory system of an affected person, so as to attempt to improve the wearer's muscle tone, flexibility and/or strength without physical manipulation of the affected limb by a physiotherapist. For example, electrodes may be placed on the surface of a wearer's skin to stimulate the sensory nerves (afferents) in the skin.

US2013/018289 describes a post-stroke stimulation device including a garment, such as a glove, to be applied to a portion of a body of a stroke patient to be treated, such as the hand. The post-stroke stimulation device includes at least one tactile actuator adapted to supply tactile stimulation to the skin of the stroke patient. Examples of such actuators include pneumatic pads that produce a jet of air or light pressure when inflated, or small rollers operable to apply gentle pressure as they are turned. The tactile actuators are controlled to be activated and deactivated in a random pattern or a pattern selected by a medical professional, so as to apply gentle stimulation to varying locations on the portion of the body being treated.

WO2008/088985 describes a neuro-stimulation system for stroke patient therapy that employs a stimulator, which may include electrode devices and/or vibration elements. In response to an electrical signal, the stimulating elements deliver electrical and/or mechanical stimulation to a body part of a wearer. The stimulation may be a subthreshold stimulation, i.e. a stimulation that is insufficient to activate the sensory cells of the wearer, but which is thought to bias those cells towards activation.

WO2019134033 A2 describes a system constructed from: EMG electrodes, EMS actuators, and EMU and altimeter sensors. The EMG electrodes measure EMG of the calf and plantarflexor muscles. The same electrodes can be used for EMS of the calf muscle. EMS actuators are for dorsiflexor muscles and plantarflexor muscles. IMU and altimeter sensors are located on each sock for detecting steps, cadence and calories burned during walking, running, cycling and other exercises. Features are extracted from the EMG signal and are compared with the features from the previous EMG test and also with the normal standard. An improvement in a condition is estimated to give feedback to the person for adjustment of therapy and other rehabilitation measures.

US2014142652 A1 describes apparatus and methods of use for treating a subject in need of assistance with breathing. In some examples shown, the subject suffers from airflow obstruction. In some other examples show, the subject suffers from chronic obstructive pulmonary disease.

It has been shown that targeting the muscle spindle afferents of a patient (i.e. nerve afferents deep within a muscle of the patient) is an effective method of treating and preventing weakness. However, the majority of stimulation systems currently proposed for treatment of post-stroke weakness are only capable of stimulating nerve afferents within the skin of a wearer.

### Summary of the invention

The invention provides a stimulation system according to claim 1. Embodiments of the invention are defined in the appended claims. For better understanding of the invention, this disclosure provides also further aspects, which may not fall under the scope of the claims.

According to one aspect, there is provided a stimulation system for a wearable article, the stimulation system comprising an actuator controllable to deliver a mechanical stimulus to a muscle of a wearer of the article, and one or more electrodiagnostic sensors, such as electromyography sensors, operable to sense muscle activity from a wearer. The actuator may deform so as to deliver the mechanical stimulation, or the actuator may move so as to deliver the mechanical stimulation, or a combination of both.

The actuator may be a piezoelectric actuator. The piezoelectric actuator may comprise a tubular structure or a part-tubular structure. The part-tubular structure may be a semi-cylinder, or a semi-cylinder with a varying radius. The tubular / part-tubular structure may be formed of piezoelectric nanofibers, and may be a piezoelectric nanofiber mesh. The structure may be knitted.

The piezoelectric actuator may further comprise a first electrode and a second electrode. The first electrode may be located on an inside of the tubular or part-tubular structure and the second electrode is located on an outside of the tubular or part-tubular structure. The inside of the structure may refer to the concave side of a part-tubular structure. The outside of the structure may refer to the convex side of a part-tubular structure.

The first electrode may comprise a first tubular electrode structure, which may be formed of conductive nanofibers, such as a conductive nanofiber mesh. The second electrode may comprise a second tubular electrode structure, which may be formed of conductive nanofibers, such as a conductive nanofiber mesh. The first tubular electrode structure may be located at least partially inside the second tubular electrode structure and may be offset from the second tubular electrode structure. The first and second tubular electrode structures may be knitted. Rather than being tubular, the first and second electrodes may be part tubular.

Alternatively, the actuator may comprise an electromagnetic actuator such as a solenoid or voice coil.

According to the invention, the stimulation device further comprises a casing enclosing the actuator. The casing may be a hard casing, and may be insulating (i.e. not conductive). The casing may enclose one or more electrodiagnostic sensors, such as electromyography sensors. The casing may comprise one or more sensors. The casing may comprise one or more EMG sensors. One or more EMG sensors may be external to the casing. The EMG sensor(s) may be (surface EMG) sEMG sensors. The casing may comprise one or more (electrocardiogram) ECG sensors. One or more ECG sensors may be external to the casing. The casing may comprise one or more (electroencephalogram) EEG sensors. One or more EEG sensors may be external to the casing.

The casing may comprise an indentation region, which may be operable to indent a muscle of a wearer.

The actuator may be operable to tap against an interior of the casing at least at the indentation region when the actuator moves and/or deforms to deliver the mechanical stimulus.

The stimulation device or system may further comprise at least one conductive plate between the actuator and the casing. The casing may be a flexible casing.

The casing may be shaped as a bead. The casing may have rotational symmetry about a longitudinal axis. Alternatively, the casing may be asymmetrical and may comprise a flattened region on an opposing side to the indentation region.

The casing may be shaped to indent into a muscle of a wearer, and may be generally ellipsoid or part ellipsoidal (e.g. ellipsoidal with a flattened region) in shape. The casing may comprise a ridge, such as an annular or part-annular ridge, which may be located at an indentation region of the casing. The casing may comprise a first end and a second end, and the ridge may be provided midway between the ends of the casing.

The casing (or at least an indentation region thereof) may be internally tubular or part-tubular, so as to cooperate with the shape of a tubular or part-tubular actuator that is received therein.

The casing may comprise one or more thread holes through which yarn is threadable to permit connection of the casing to the wearable article. In the case of a bead having a flattened region, the thread holes may be adjacent the flattened region.

The casing may be configured to clip around a yarn to permit connection to a wearable article. The casing may comprise a first part and a second part, which may be hingedly connected together. Each of the first and second parts may comprise one or more recesses configured to align when the casing is closed, so as to define one or more thread holes.

The casing may comprise a plurality of parts. The casing may comprise a first part (which may be configured to attach to a garment), and a second part which is configured to move relative to the first part to deliver mechanical stimulation to a muscle of a wearer.

The stimulation device or system may comprise at least one, for example two, electrical connectors operable to connect the first and second electrodes to a power source.

The stimulation device or system may further or alternatively comprise any of the features of the specific examples described below in the detailed description section.

According to a second aspect we provide a wearable article comprising a stimulation device or system for a wearable article. The stimulation device may comprise a piezoelectric actuator controllable to deform so as to deliver a mechanical stimulus to a muscle of a wearer of the article. The stimulation system may comprise an actuator controllable to deliver a mechanical stimulus to a muscle of a wearer of the article, and one or more electromyography sensors operable to sense muscle activity from a wearer.

The wearable article may comprise any of the features described above in connection with the stimulation device and/or system, and/or any features selected from the detailed description below.

The wearable article may comprise one or more electromyography sensors operable to sense muscle activity from a wearer. One or more of the EMG sensors may be comprised in or located within the casing. The wearable article may comprise a power source operable to supply an electrical current to the stimulation device and/or to the one or more electromyography sensors. The power source may be comprised in or located within the casing.

The power source may comprise one or more energy harvesting yarns and may further comprise at least one capacitor operable to store electrical energy harvested by the one or more energy harvesting yarns. The energy harvesting yarns may comprise piezoelectric nanofibers. The wearable article may comprise two layers, and the energy harvesting yarns may be located between the two layers such that the skin of a wearer is not in contact with the energy harvesting yarns, and/or such that the energy harvesting yarns are not visible on an exterior of the garment.

The wearable article may further comprise a controller operable to control the stimulation device to deliver the mechanical stimulus to the muscle of the wearer. The controller may be operable to receive an electromyography signal from the one or more electromyography sensors, and the controller may be operable to deliver the mechanical stimulus in response to the received electromyography signal. The controller may be comprised in or located within the casing. Alternatively, the controller may be located externally of the casing, for example elsewhere in the wearable article and/or in another/other bead(s) shaped to contain the controller parts. Alternatively, or additionally, the wearable article may comprise a switch, and the controller may be operable to deliver the mechanical stimulus in response to a wearer input, such as an operation of the switch.

The wearable article may be shaped to be worn adjacent a selected muscle stimulation site, and the stimulation device may be located at a selected location on the wearable article so as to deliver mechanical stimulation to the selected muscle stimulation site when the wearable garment is worn by the wearer. "Worn by the wearer" means, in this context, worn in the manner intended by the designer, for example, such that the wearable article is the correct size to fit the wearer (e.g. not too big, and hence too loose, or not too small, so as to change the relationship between the selected location and the selected muscle stimulation site).

The wearable article may be a garment. The stimulation device may be located at a selected location on a sleeve of the garment so as to deliver mechanical stimulation to a selected muscle stimulation site in an upper limb of the wearer. The selected muscle stimulation site may be a muscle in the forearm. The stimulation device may be located at a selected location below the elbow of the sleeve of the garment. The stimulation device may be located at other selected positions in the garment so as to deliver the stimulus to a selected muscles stimulation site in another area of the body.

The wearable article may include a first region of having a first tension and a second region having a second tension, wherein the selected location is provided in the first region and the first tension is higher than the second tension. For example, the first region may comprise an elbow region of a sleeve of the garment. The second, lower tension region may be provided at the head of the sleeve and/or at a shoulder and/or at a body portion of the garment. The first region may comprise alternative yarn combinations to increase its compressive strength. This may increase the contact between the bead and/or the EMG sensor(s) with the body.

The wearable article may further or alternatively comprise any of the features of the specific examples described below in the detailed description section.

According to a third aspect we provide a method of manufacturing a piezoelectric actuator, the method comprising knitting one or more piezoelectric nanofiber yarns into a tubular mesh to produce a piezoelectric tubular or part-tubular structure.

The method may comprise electrospinning the one or more piezoelectric nanofiber yarns, and may further comprise coating the piezoelectric nanofiber yarns.

The method may comprise twisting one or more piezoelectric nanofiber yarns together, for example twisting 2, 3, 4, 5, 6, 7 or more piezoelectric nanofiber yarns together prior to knitting the tubular mesh.

The method may further comprise knitting one or more conductive nanofiber yarns into respective tubular meshes to produce first and second tubular or part-tubular electrode structures. The first tubular or part-tubular electrode structure may be knitted inside the piezoelectric tubular or part-tubular structure and the second tubular or part-tubular electrode structure may be knitted outside the piezoelectric tubular or part-tubular structure, such that the piezoelectric tubular or part-tubular structure is located between the first and second tubular or part-tubular or part-tubular electrode structures.

The method may further comprise knitting one or more conductive nanofiber yarns into alternative shapes. The nanofiber yarns may be knitted into meshes which are non-tubular.

Knitting may comprise varying the tension of the nanofiber yarn such that the tension is lower at the centre of the tubular structure than at the start and end of the tubular structure.

The method may further comprise offsetting the first and second electrode structures with respect to the piezoelectric tubular structure.

The method may further comprise locating the piezoelectric actuator within a casing, and optionally locating one or more conducting plates between the piezoelectric actuator and the casing.

The method may comprise connecting the first and second tubular electrode structures to a power source.

The method may further or alternatively comprise any of the features of the specific examples described below in the detailed description section.

According to a fourth aspect, there is provided a non-claimed method of delivering a stimulus to a wearer. The method may comprise providing a wearable article comprising a stimulation device or stimulation system, and delivering a stimulus to a muscle of the wearer using the stimulation device or stimulation system. The method may be performed using the stimulation devices or systems or wearable articles described herein.

The devices and methods described herein may be capable of stimulating the muscle afferents of a patient, such as a patient affected by post-stroke limb weakness. It will be appreciated that such a system and method may have utility in treating other forms of limb weakness or spasticity, such as that present in other neurological disorders such as cerebral palsy. The system and method may also find utility in providing muscle stimulation to other wearers, for instance to improve circulation and/or lymphatic drainage and in general for muscle training for sports performance.

### Brief description of the drawings

The invention will now be described in more detail, by way of example only, with reference to the following drawings:
**Figure 1** schematically shows a muscle stimulation device, and in particular shows: (a) a cross-section through the device, (b) an end view of the device, and (c) a side view of the device;
**Figure 1a** shows further detail of a component within the muscle stimulation device of Figure 1;
**Figure 2** shows (a) a schematic cross section of a piezoelectric actuator, and (b) an image showing the structure of a piezoelectric mesh;
**Figure 2a** schematically shows an exemplary knitted structure;
**Figure 3** shows a cross section through a twisted nanofiber yarn;
**Figure 3a** shows a selection of alternative yarn structures;
**Figure 4** illustrates an example electrospinning arrangement;
**Figure 5** shows nine possible shapes for a casing of a stimulation device;
**Figure 6** schematically illustrates a garment including a muscle stimulation device;
**Figure 7** shows (a) an image illustrating electrical connections in a garment, and (b) a diagram schematically illustrating electrical connections to a muscle stimulation device;
**Figure 8** illustrates an example garment pattern including layout of electrical components;
**Figure 9** shows an interior view of an exemplary garment front panel showing conductive tracks at the neckline, as well as a close up view of said conductive tracks;
**Figure 10** shows (a) interior view of a first exemplary garment sleeve panel showing conductive tracks and (b) an exterior view of the same sleeve panel;
**Figure 11** shows an interior view of a second exemplary garment sleeve panel showing conductive tracks;
**Figure 12** illustrates example structures for (a) energy harvesting yarn, (b) a supercapacitor, and (c) an electromyography (EMG) sensor and switch;
**Figure 13** illustrates alternative example garment sleeve head patterns;
**Figure 14** illustrates another example garment pattern;
**Figure 15** illustrates a further example garment pattern;
**Figure 16** illustrates an example sleeve structure at the top of the sleeve; at the bottom of the sleeve (Figure 17).
**Figure 17** illustrates an example sleeve structure at the bottom of the sleeve;
**Figure 18** illustrates an example sleeve structure including a sensor;
**Figure 19** shows an example of diverging conductive tracks within a sleeve;
**Figure 20** shows examples of alternative stimulation systems;
**Figure 20A** shows an alternative stimulation device having a knitted actuator;
**Figure 20B** shows a further alternative stimulation device having a composite actuator;
**Figure 20C** shows alternative casing designs;
**Figures 21A and 21B** show further alternative casing designs;
**Figure 22** shows another alternative casing design for a stimulation device;
**Figure 23** shows more alternative casing designs for a stimulation device;
**Figure 23A** shows further detail of some of the casing designs of Figure 23;
**Figures 24A and 24B** show alternative structures for a garment;
**Figure 25** shows a section of a sleeve structure for a garment;
**Figure 26** shows example actuators;
**Figure 27** shows further example actuators; and

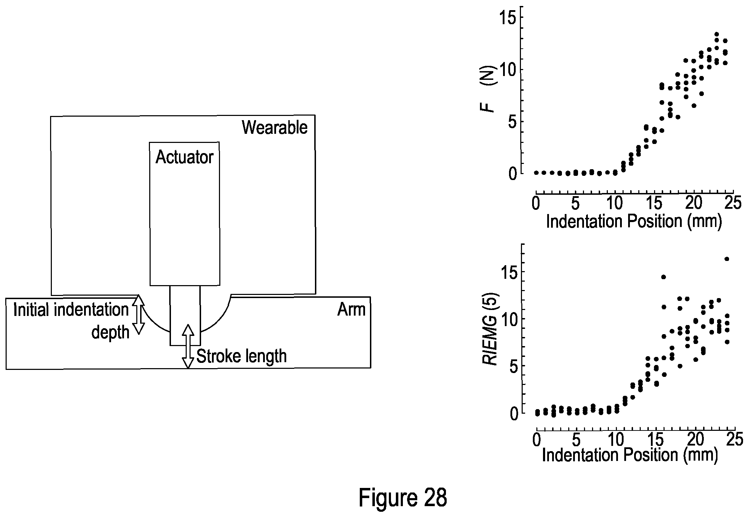

**Figure 28** shows a graph of how actuator force affects indentation.

### Detailed description

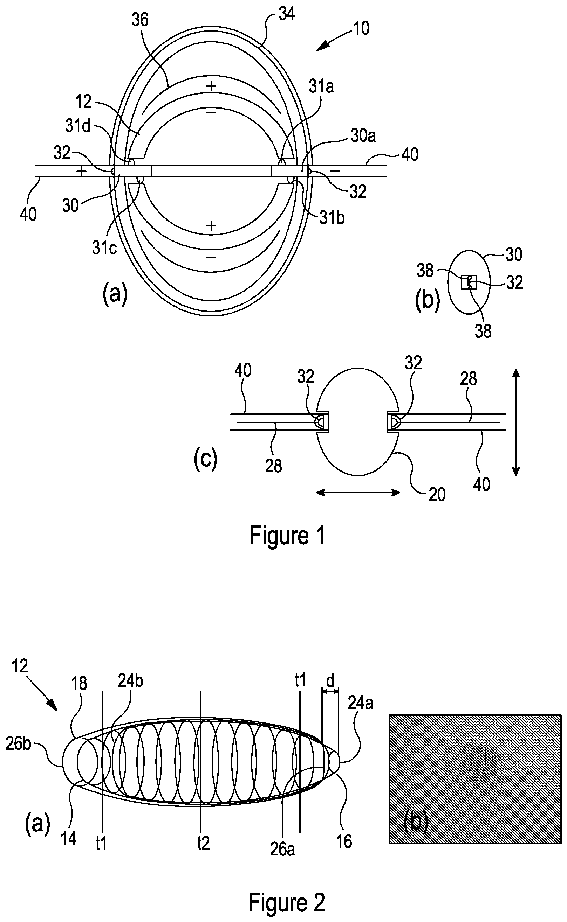

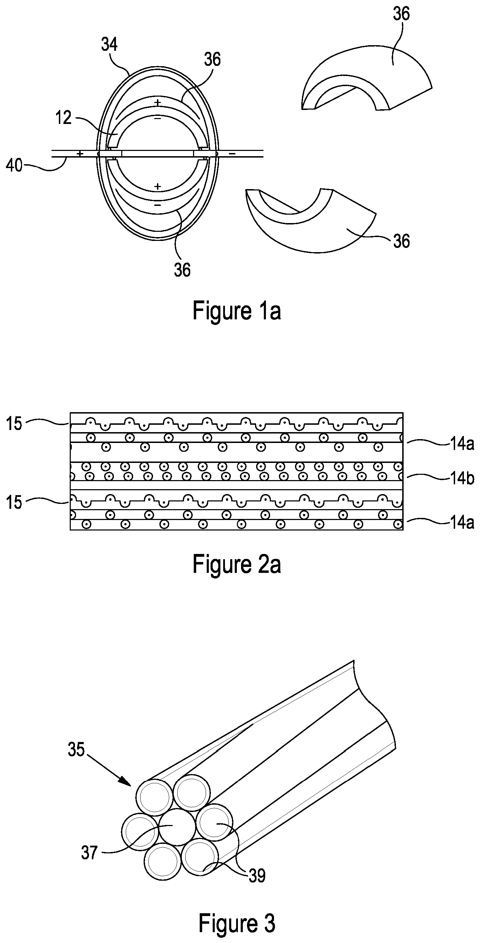

Figure 1 shows a stimulation device 10 for a wearable article. The stimulation device comprises a piezoelectric actuator 12 that is controllable to deform so as to deliver a mechanical stimulus to a muscle of a wearer of the article.

The piezoelectric effect refers to the ability of some materials to acquire an electric charge on the application of mechanical stress. When a mechanical stress is applied to a piezoelectric material, the crystal structure of that material is caused to polarise, resulting in a charge developing between opposing sides of the material, so causing a current to flow. The piezoelectric effect is reversible, in that application of a current to a piezoelectric material has the opposite effect of causing a mechanical stress in the material, which can result in deformation of that material. Piezoelectric materials are thus operable to deform (i.e. to change physical shape) when an electric current is applied to such a material.

The piezoelectric actuator 12 shown in Figure 1 is shaped to deform to an extent sufficient to deliver a mechanical stimulus to the muscle afferents of a wearer of the article when an electric current is applied to the actuator 12. Such a stimulus is referred to herein as a "tap".

To be effective in providing a stimulus to the muscle afferents of a wearer, rather than simply to the surface of the wearer's skin, the stimulation device 10 is shaped to indent the flesh of a wearer's limb. To this end the stimulation device includes a casing 34 (discussed in more detail later) which substantially encloses the piezoelectric actuator. The piezoelectric actuator 12 is operable to deform by an amount sufficient to tap against the casing of the stimulation device. The force generated by the tap is thus delivered to a desired location in a muscle, tendon or nerve of the wearer by the shaped casing.

The piezoelectric actuator 12 is shown in more detail in Figure 2, in which it can be seen that the piezoelectric actuator 12 includes a piezoelectric structure 14 that is substantially tubular in shape. That is, the piezoelectric structure 14 is formed from a tube of piezoelectric material defining a hollow interior. The tube of piezoelectric material shown in Figure 2 is formed of a piezoelectric nanofiber mesh. An example image of a structure of such a mesh is shown in picture (b) of Figure 2.

The piezoelectric actuator 12 further includes a first electrode 16 and a second electrode 18. The electrodes are operable to supply an electrical current to the piezoelectric structure 14, in order to cause the piezoelectric material to deform in accordance with the reverse piezoelectric effect.

The first electrode 16, which in the example shown is formed of a conductive nanofiber mesh, is located on an inside of the piezoelectric tubular structure 14. The second electrode 18, which in the example shown is also formed of a conductive nanofiber mesh, is located on an outside of the piezoelectric tubular structure 14.

Figure 2a schematically shows one exemplary knitted structure, in which the piezoelectric structure 14 is knitted as a partial interlock 14a, tubular 14b structure with tuck spacer 15 to connect the inner and outer electrodes (not shown) to the piezoelectric structure 14.

Both electrodes 16, 18 are, like the piezoelectric structure, substantially tubular, and each has respective first and second ends. The piezoelectric actuator 12 thus includes three nested tubular layers: a first, inner, layer, which includes the first electrode 16; a second, outer, layer, which includes the second electrode 18; and a third, middle, layer, which includes the piezoelectric structure 14, and which is sandwiched between the two electrodes. In the example shown all three layers are of generally ellipsoid shape and all are formed from nanofiber mesh.

A casing 34 encloses the piezoelectric actuator 12. The casing provides a hard surface for the piezoelectric actuator to tap against when the piezoelectric actuator 12 deforms. The casing is not conductive, in order to shield the wearer of the article from the conductive parts of the device (i.e. electrodes, piezoelectric structure). The casing may be formed from any non-conductive material that is hard enough to hold its shape, such as carbon fiber, nylon, elastomeric polyurethane or flexible polyurethane resin. The casing is shaped so that when the stimulation device is incorporated into an article, such as a garment, that is intended to be worn closely against the skin of the user (as discussed in further detail below), at least portion of the casing indents into the flesh of a wearer when the article is correctly worn (i.e. worn as intended). The indentation portion of the casing preferably indents at least 1mm into the wearer's flesh, for example between 1mm and 20mm, or between 1mm and 16mm, in order to deliver the force of the tap into a wearer's muscle.

The nanofiber meshes shown in Figure 2, both electrodes and piezoelectric structure, may be constructed from one or more nanofibers. Suitable nanofibers may be created using any appropriate nanofiber formation method, such as electrospinning from a polymer solution or polymer melt having the desired piezoelectric and/or conductive properties.

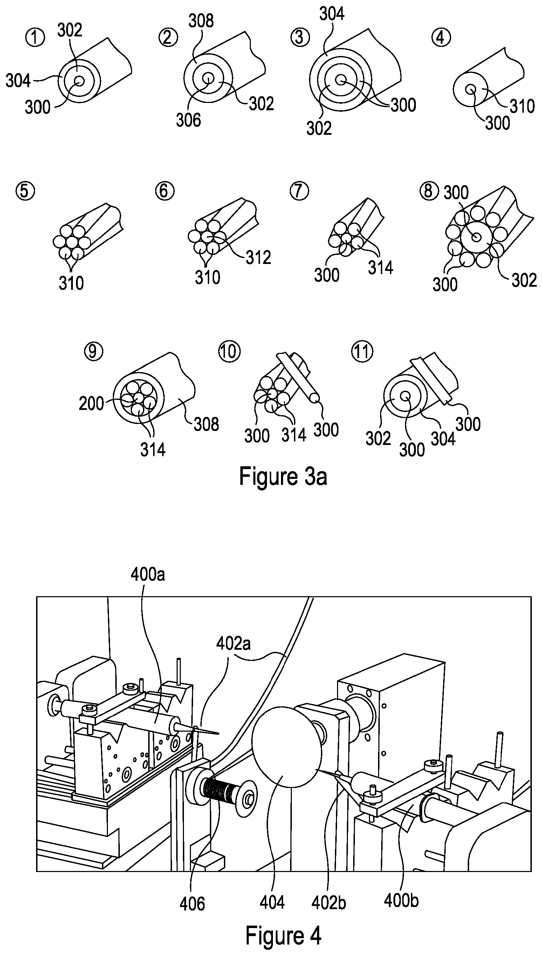

Early samples used a traditional method of electrospinning to deposit PVDF nanofibers (from solution) directly onto a conductive yarn substrate. However, this produced much waste PVDF nanofibers and was difficult to scale up. Therefore, a pull and twist electrospinning method (using a metal funnel and yarn spool set up - see Figure 4) was subsequently used. Not only does this enable the production of continuous nanofiber yarn formation (meterage of yarn depends on amount of solution contained in the syringes - therefore also reliant on syringe size), but this method appears to produce a greater piezoelectric response. The twisting and pulling of the nanofibers from the metal funnel cone seemingly aligns the nanofibers, improving their orientation. In some studies, this method has been seen to produce effective piezoelectric properties without the need for poling, using a co-polymer of PVDF; PVDF-TrFe.

Indeed, techniques such as stretching, fiber formation at given temperatures including melt extrusion or the combination of stretching and poling may be employed to increase the β- phase content of such polymers. Electrospinning is the most effective method due to what has been termed "high stretching forces exerted on electrified solution jets".

In an exemplary electrospinning process electrospinning solution held within two syringes 400a, 400b is released at a defined flow rate. Whilst this is happening, the solution is polled by a high voltage meter (not shown) which is connected to each syringe needle by a high voltage connection 402a, 402b terminating in (in this example) a crocodile clip attached to a needle of each respective syringe. The voltage applied can be varied and thus yield different results (and in particular impacts the orientation of the dipoles and the piezoelectric output of the yarn). Nanofibers are deposited into a rotating metal funnel cone 404, and pulled and twisted therefrom onto a rotating yarn spool 406. The distance of the syringe tip to the metal funnel cone 404 (tip to collector distance) and the rotating speed of the collector (metal funnel cone 404) as well as the yarn spool 406 can all be changed. Each of these variables has a direct impact on the quality of yarn produced.

Two examples of suitable electrospinning parameters are set out in Table 1, below, for two different solutions.

### Formula:

### Solef 11008 & 31508; 0.1wt% graphene

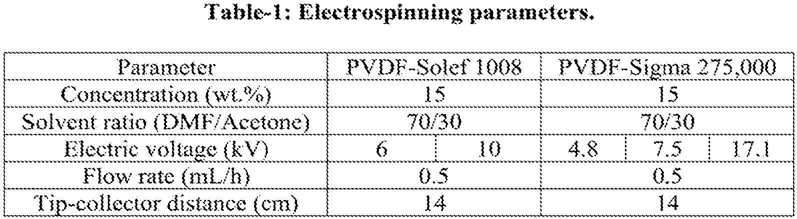

Both solutions include a proportion of a piezoelectric material, such as polyvinylidene fluoride (PVDF). Other piezoelectric materials could be used.

A number of samples were created from the above solutions, detailed in Table 2 below. The sample nanofibers were created with the modified electrospinning technique discussed above.

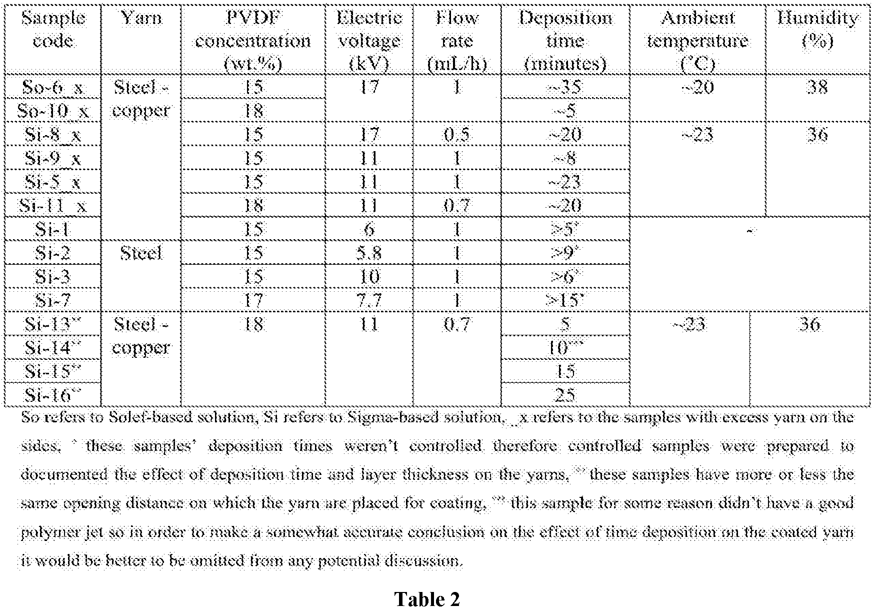

The use of a metal funnel cone and spool permits the integration of conductive particles, such as silver or graphene, into the PVDF whilst spinning a continuous nanofiber yarn, as discussed above. The amount of filler material that may be integrated differs depending on the particle to be integrated. In the case of using graphene, the maximum saturation point for using graphene as a filler is currently 0.1wt%. Alternatively, NiO/SiO2 nanoparticles from 0-16wt% have been seen to increase the piezoelectric effect. Further alternatively, Ag particles may be integrated into the yarn to increase piezoelectric effect from 3wt%.

Polarisation within a piezoelectric material occurs as a result of interfacial space charge which happens at electrodes and at heterogeneities such as grain boundaries. This can be used to suggest an increase in beta phase fraction occurs as a result of the alignment of the graphene nanoparticles in the 'all trans' (TTT) conformation: The electrostatic interaction between the surface charge of the nanoparticles and the dipoles present in the carbon backbone of the polymer matrix is thought to play a key role in promoting this alignment.

In comparison to 'neat' PVDF, the addition of graphene sees the shifting of the -CH2- vibrational bands towards the lower frequency region, confirming the presence of interfacial interaction between the -CH2-dipole and graphene nanoparticles.

Increasing the filler concentration (graphene) increases the shifting of peak position due to the dampening oscillation of the -CH2- dipoles.

Increasing the graphene content increases the transformation of alpha phases of 'TGTG' conformation to beta phases of 'TTT'; later aggregating locally ordered beta crystals to form beta crystals, considered necessary to enhance the piezoelectric property. The addition of further fillers aggregates all the beta crystals into beta polymorph from alpha polymorph until the point of saturation, which is observed at 1 wt% within this study.

Any of the nanofibers noted in the table above may be suitable for constructing the piezoelectric structure 14. For example, sample (Si-3) was used to create the example piezoelectric knitted structure imaged in picture (b) of Figure 2, with the addition of 0.1% wt graphene.

The electrodes 16, 18 are not constructed from a piezoelectric nanofiber but could for example be constructed from any conductive nanofiber yarn, such as a stainless steel yarn, e.g. 100% inox conductive yarn. Alternative conductive yarns include copper, and/or silver, and/or composite yarns such as a yarn including 97% inox / 3% copper; or 97% silver / 3% nylon.

The resulting nanofiber yarn may be coated, e.g. dip coated, in a coating to improve strength and/or durability, such as with polyvinyl butyrol (PVB). Silver particles may be included within the coating to improve delivery of charge from the outer electrode mesh to the inner piezoelectric (e.g. PVDF/ graphene) mesh. PVB (with Ag particles) can thus be used to secure the nanofibers and reduce degradation rates in response to tensile stresses, such as those occurring during wearing and washing.

A plurality of nanofiber yarns may be combined together to improve the strength and/or increase the diameter of the final yarn. An example of such a composite nanofiber yarn 35 is shown in Figure 3. The composite yarn 35 is formed from a plurality of nanofibers twisted together, in particular seven nanofibers twisted together. A core nanofiber yarn 37 may be untwisted. A plurality of further nanofiber yarns 39 (six are shown) may be twisted around the core nanofiber in a generally spiral manner. A measure of weight/thickness of yarn is dtex. The yarn shown in Figure 3 was twisted until the yarn achieved a dtex of at least 200, e.g. 220.

All the nanofiber yarns in such a composite yarn 35 may have the same composition, or may have different compositions. The composite yarn shown in Figure 3 has a core of conductive material, e.g. a metal, such as steel, and in this particular example is composed of 100% inox yarn. The surrounding nanofiber yarns are composed of twisted electrospun PVDF nanofibers having a 0.1wt% graphene particle filler, which are dip coated in a PVB/Ag encapsulation layer. In particular, the nanofibers may be electrospun from sample (Si-3) in Table 2 above but with the addition of 0.1% wt graphene. It will be appreciated that more or fewer nanofibers may be twisted together if required.

Figure 3a shows a plurality of other possible composite nanofiber yarn structures. The constructions of the depicted yarn constructions are outlined in Table 3, below. It will be appreciated that these are example yarn structures only, and that other piezoelectric yarn structures may be used in creation of a piezoelectric structure 14 of the type discussed herein.

**Table 3**

| **Yarn** | **Components** |
|---|---|
| Structure 1 | Core: Inox yarn (300) |
| | Inner: Electrospun PVDF nanofibers (302) |
| | Outer: Dip coated PVB (304) |
| Structure 2 | Core: Cotton-ZnO yarn, e.g. Perma antibacterial yarn (306) |
| | Inner: Electrospun PVDF nanofibers (302) |
| | Outer: Dip coated PVB / Ag (308) |
| Structure 3 | Core: Inox yarn (300) |
| | Inner 1: Electrospun PVDF nanofibers (302) |
| | Inner 2: Inox coating (300) |
| | Outer: Dip coated PVB (304) |
| Structure 4 | Core: Inox yarn (300) |
| | Outer: Electrospun PVDF nanofibers with Ag particles (310) |
| Structure 5 | Multiple strands of twisted electrospun PVDF nanofibers with Ag particles (310) |
| Structure 6 | Core: Copper yarn (312) |
| | Outer: Multiple strands of twisted electrospun PVDF nanofibers with Ag particles (310) |
| Structure 7 | Core: Inox yarn (300) |
| | Outer: Multiple strands of twisted melt-spun PVDF monofilament (314) |
| Structure 8 | Core: Inox yarn (300) |
| | Inner: Electrospun PVDF nanofibers (302) |
| | Outer: Multiple strands of twisted Inox yarn (300) |
| Structure 9 | Core: Inox yarn (300) |
| | Inner: Multiple strands of twisted melt-spun PVDF monofilament (314) |
| | Outer: Dip coated PVB / Ag (308) |
| Structure 10 | Core: Inox yarn (300) |
| | Outer: Multiple strands of twisted melt-spun PVDF monofilament (314) |
| | Second yarn: Inox yarn (300) |
| Structure 11 | Core: Inox yarn (300) |
| | Inner: Electrospun PVDF nanofibers (302) |
| | Outer: Dip coated PVB (304) |
| | Second yarn: Inox yarn (300) |

A tubular nanofiber mesh, such as the meshes which form the piezoelectric actuator 12 described above, may be created from a nanofiber yarn, such as the composite nanofiber yarn 35. One method for creating such a mesh is to knit a continuous circular tube, for example using a knitting machine. A machine such as a 'Multi gauge Stoll CMS ADF 32 W' may be used. Other machines with a similar or finer gauge may also be used.

The tension of the yarn may be set higher at the start of the knit, reduced towards the centre of the knit, and increased again towards the end of the knit. Decreasing the tension in the knit has the effect of increasing the diameter of the resulting knitted tube, thus decreasing the tension toward the centre of the tubular structure can result in a generally ellipsoid shape. Referring again to Figure 2, the tubular shape of the piezoelectric structure 14 and electrodes 16, 18 shown therein may be created using a tension of 8 at the start and the end of the knit (locations t₁) and a tension of 10.5 at the centre of the knit (location t₂) in the knitting machine mentioned above. It will be appreciated that tension can vary slightly between machines, and so other tensions may be selected depending on the machine which is used. The tension may vary gradually between these locations to ensure a smooth shape.

One problem faced by the applicants in designing a muscle stimulation device that is operable to indent a muscle of a wearer, rather than just the surface of the wearer's skin, was ensuring the device is operable to deliver taps of sufficient force (e.g. 6-10N). It has been found that a piezoelectric mesh of the type described above is able to deliver taps of sufficient force. The ellipsoid shape described above has been found to be particularly useful in delivering a large indentation. A shape of the type described above may be operable to deform by up to 2mm, for example between 1mm and 2mm when a charge is applied, which may result in a force of between 6 and 10N being transmitted to the casing of the device, which in turn transmits the force of the tap into the muscle of a wearer. The amount of deformation, and hence the force of the tap, may be increased by providing additional piezoelectric layers.

The first and second electrodes each comprise a respective pair of electrical connections 24a, 24b and 26a, 26b. Using the electrical connections, the stimulation device may be connected to a power source (not shown) via one or more conductive yarns 28.

In the example shown, the first electrode is offset from the second electrode by a separation distance d. This offset causes the first end of the first electrode to extend out from within the interior of the second electrode. A first electrical connection 24a to the first electrode 16 is provided on that protruding first end, and a second electrical connection 24b to the first electrode is provided on the opposing, nested, second end. Conversely, a first electrical connection 26a to the second electrode is provided on a first end of the second electrode, and a second electrical connection 26b to the second electrode 18 is provided on the opposing end of the second electrode. Offsetting the electrodes improves the ease of access to the electrical connections.

A conductive connector 30a may be electrically connected to the first electrical connection 24a of the first electrode 16 via a first connection loop 31a and to the first electrical connection 26a of the second electrode 18 via a second connection 31b. Similarly, a conductive connector 30b may be electrically connected to the second electrical connection 24b of the first electrode 16 via a third connection loop 31c and to the second electrical connection 26b of the second electrode 18 via a fourth connection loop 31d. The conductive yarn 28 may then be connected to a respective contact loop 32 of each connector 30 in order to connect the stimulation device 10 to a power source. The connectors may be formed of any suitably conductive material, such as silver.

As discussed above, the piezoelectric actuator 12, including piezoelectric structure 14 and electrodes 16, 18, is enclosed in a casing 34. The casing 34 is not conductive, in order to shield the wearer of the article from the conductive parts of the device (i.e. electrodes, piezoelectric structure). Conductive areas of the stimulation device which are exposed through the casing (e.g. portions of the connectors 30 and/or portions of electrodes 16, 18) in order to connect to the conductive yarn 28 may also be insulated. For example, an insulating coating may be applied over the conductive portions of the stimulation device after the piezoelectric actuator is enclosed within the casing.

One or more intermediate plates 36 may be located between the piezoelectric actuator and the casing 34. The intermediate plate(s) 36 are formed of a deformable material, in this case a conductive material such as steel, and can assist in enhancing the force of the tap. The intermediate plate 36 may be free floating within the stimulation device, such that it is pushed outwards and/or deformed by the deformation of the piezoelectric structure. Figure 1a shows an example stimulation device 10 which includes two curved intermediate plates 36, which are also shown in perspective view for clarity. Each intermediate plate 36 is shaped to fit between the piezoelectric actuator 12 and the casing 34 on opposing sides of the stimulation device. To this end the intermediate plates have a generally C shaped cross section, the diameter of which tapers towards the ends to mimic the cross section of the piezoelectric actuator 12.

It will be appreciated that more than two intermediate plates may be provided if required. It will further be appreciated that only one intermediate plate may be provided, for example on one half of the stimulation device, if it is not required for the tap to be symmetrical.

At least one thread hole 38 is provided in the stimulation device through which a base yarn 40 may be threaded. The thread hole 38 allows the stimulation device 10 to be connected to a wearable article, as described in more detail below. The base yarn 40 may be any yarn from which a wearable article may be constructed, such as cotton, silk, viscose, polycotton, wool, and may be a stretchable yarn, e.g. a yarn having a proportion of lycra or elastane. The yarn may be impregnated with an antibacterial agent, such as zinc oxide. This would provide a reduced need to wash the garment due to the antibacterial and anti odour properties. This is especially beneficial for a garment used for stroke rehabilitation, where mobility issues can affect activities of daily living (ADLs) including washing clothes. Reducing the need to wash the garment whilst maintaining optimum hygiene standards is therefore useful at home, and also in cases where the garment may be used in hospital.

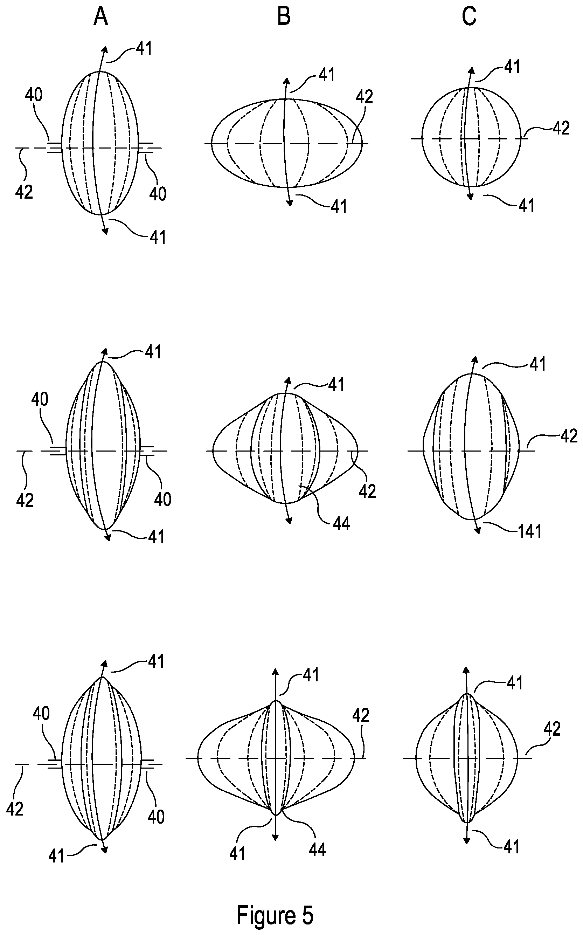

The stimulation device 10 preferably has a curved outer surface, avoiding sharp edges which might cause pain during delivery of the tap. The stimulation device may be generally ellipsoid in shape, to give the overall appearance of being a bead. Examples of nine such beads are shown in Figure 5. Each bead has one or more indentation portion 41, being the portion(s) of the bead that are shaped to press into the flesh of a wearer when the stimulation device is incorporated into a wearable article. The beads shown all have at least one pair of diametrically opposed indentation portions which have substantially the same shape, to ensure an equal indentation regardless of the orientation of the bead on the base yarn(s) 40.

As in the examples shown, the stimulation device 10 has rotational symmetry about a longitudinal axis 42, which may be a thread axis falling between the one or more thread holes 38. The shape of the stimulation device is selected to control the amount of indentation caused by the stimulation device. For example, an ellipsoid having its long axis perpendicular to the thread axis, as is the case for the beads in column A, will cause more indentation than a substantially spherical bead, as shown in column C. The beads in both columns B and C will all cause more indentation that an ellipsoid having its long axis parallel to the thread axis, as is the case for the beads in column B. The amount of indentation due to the stimulation device may thus be controlled by selecting a size of the dimension of the stimulation device that is perpendicular to the thread axis. The dimension may be a radius perpendicular to the thread axis, and the size of the radius may be between 1mm and 20mm, for example between 1mm and 16mm, between 1mm and 10mm, or between 1mm and 5mm.

In the examples shown the axis of rotational symmetry 42 substantially coincides with the thread axis, as well as with the contact points 32 for the conductive yarn, each of which is disposed between a respective pair of thread holes 38. Rotational symmetry further helps to ensure that the strength of the tap that is delivered is independent of the orientation of the stimulation device when it is sewn into a wearable article.

The first row of Figure 5 shows two substantially ellipsoid beads and one substantially spherical bead. The second and third rows show similar ellipsoid beads, which further include an annular ridge 44 about a widest part of the ellipsoid. The annular ridge may increase the strength of the tap delivered by the stimulation device, by concentrating the force of the tap into a smaller surface area at the indentation portion.

A stimulation device 10 of the type described above with respect to Figures 1 to 5 may be sized to have a largest diameter between 2mm and 20mm, for example between 3mm and 10mm. Such devices may be included in a wearable article 50.

The wearable article 50 is shaped to be worn adjacent a selected muscle stimulation site 51 on a limb of a wearer. The stimulation device 10 is thus located at a selected location 52 on the wearable article, such that, when the wearable garment is worn by the wearer, the stimulation device 10 is located next to the selected muscle stimulation site 51 so as to be controllable to deliver mechanical stimulation to the selected muscle stimulation site 51 when required. The wearable article may be a garment.

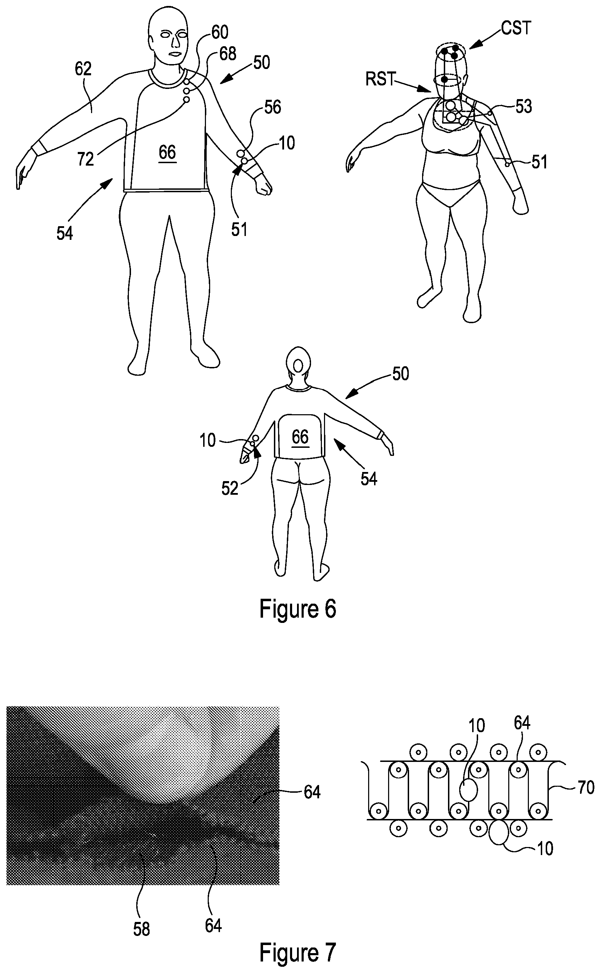

It will be appreciated that the garment into which the stimulation device is incorporated will depend on the limb to which it is required to deliver stimulation. In the example shown in Figure 6, the wearable article is a long sleeved garment 54, and the limb to be treated is an upper limb of the wearer.

The control of hand function in humans is dominated by the corticospinal tract (CST), with other pathways such as the reticulospinal tract (RST) contributing to the control of the hand. Following a corticospinal lesion, such as might result from a stroke, levels of input from the CST and RST to upper limb and hand function changes, as the RST partly subserves recovery and is especially important in motor recovery. Flexor muscles are seen to become selectively strengthened whilst extensor muscles remain weak, suggesting that the rise in input from the RST can also limit the quality of recovered movements as well as presenting a barrier to engaging in upper limb training. Prolonged non-use as a result can lead to contracture, enhanced pain, further impacting the use of the limb and the physical identity and behaviour of the body.

Within the first six weeks post injury, there is seen to be natural biological recovery and some sort of neuronal plastic changes are observed. Muscle overactivity, hyperreflexia and thus spasticity arise as a result of the process of plasticity. It is therefore considered, and has been demonstrated that, modifying levels of the RST can improve upper limb functional recovery.

In the example shown in Figure 6, stimulation may be provided to the RST via mechanical activation of muscle spindle afferents at a selected muscle stimulation site in the arm of the wearer. The selected muscle stimulation site 51 may be provided on a muscle in the lower arm of the wearer. The stimulation device may thus be located on the garment such that when the garment is worn the stimulation device is controllable to deliver a tap to a muscle adjacent an elbow of the wearer, such as one or more of the muscles that control hand function. An alternative (or additional) muscle stimulation site 53 could be selected in the upper arm of a wearer, just below the shoulder. The stimulation site 53 might be preferable if the stimulation device is incorporated into a short sleeved garment. It will be appreciated that different muscle stimulation sites could be selected, for example if it is desired to provide stimulation to a lower limb, and that those stimulation sites may be unrelated to the RST.

The wearable article 50 further includes one or more electromyography (EMG) sensors 56. EMG sensors 56 are operable to sense muscle activity. In particular, the EMG sensors 56 may be operable to sense that the wearer is moving, or attempting to move one or more of their muscles. Detection of muscle activity by the EMG sensor(s) 56 may thus be used to trigger the operation of the stimulation device.

The garment further includes a power source operable to supply electrical current to the stimulation device 10 when it is desired to deliver muscle stimulation. In the examples shown in Figures 6 to 11, the power source includes one or more energy harvesting yarns 58 and at least one capacitor 60, such as supercapacitor, operable to store electrical energy harvested by the one or more energy harvesting yarns. The energy harvesting yarns 58 may comprise a piezoelectric material, such that the energy harvesting yarn is operable to generate electricity when the wearer of the garment moves. A suitable energy harvesting yarn may be constructed from nanofibers in the same manner as is discussed above with respect to the piezoelectric structure. That is, a piezoelectric yarn that is suitable for constructing the piezoelectric structure 14 could equally be used as an energy harvesting yarn.

Energy harvesting yarn 58 may be provided in one of more energy harvesting regions 62 of the wearable article. The energy harvesting yarns may be shielded from direct contact with the skin of a wearer. This may be achieved, for example, by constructing the wearable article of two layers, and locating the energy harvesting yarn between those two layers. An example of such an arrangement is shown in Figure 7, in which energy harvesting yarn is woven between two outer layers of a non-conductive base textile 64, such as lycra.

The energy harvesting yarn may include piezoelectric nanofibers, for example composite piezoelectric nanofibers of the type described above. The nanofibers may be coated with an insulating material to protect the wearer, as well (or instead of) a coating to improve durability, as discussed above.

Not all regions of the wearable article need to contain energy harvesting yarn. The wearable article may thus comprise non-energy harvesting regions 66, which are formed solely of the base textile. The use of energy harvesting yarn as a power source promotes mobility in the body of the wearer, which can be beneficial to those suffering from stroke / lymphedema.

The wearable article additionally includes a controller 68, such as a microcontroller. The controller is operable to control the stimulation device 10 to deliver the mechanical stimulus to the muscle of the wearer. The controller is electrically coupled to the stimulation device, for example by conductive yarn 70, such as silver yarn.

A switch 72 may be provided. The switch is electrically coupled to the controller and to the stimulation device. The switch 72 may be operable by the wearer of the garment to cause the controller to activate the stimulation device. Alternatively, or additionally, the switch may be operable to disable the stimulation device.

All electrical components and connections are shielded from the wearer, for example by insulating the conductive components. It may be necessary to remove insulating coating from the conductive connections of the stimulation device before attaching electrical connections to the device. Such an insulation coating may be reapplied after connection.

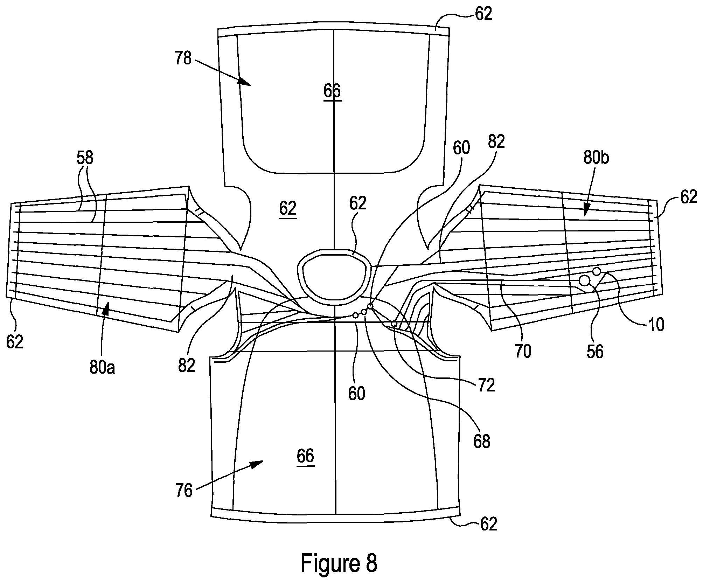

Referring now to Figure 8, a pattern 74 is shown for a long sleeved garment, such as the garment 54 of Figure 6. The pattern includes a front body portion 76, a rear body portion 78 and respective left and right sleeve portions 80a and 80a. The sleeve portions are "grown on", in that they are formed (e.g. cut) from the same piece of material as the front and back body portions. The two sleeve portions 80a, 80b are thus connected to the front and back portions 76, 78 at a sleeve head region 82 of the garment pattern. Energy harvesting yarn and/or conductive yarn is thus able to pass between the sleeves of the garment and the body portions through the connected sleeve head regions 82 without needing to be joined at a shoulder seam of the garment.

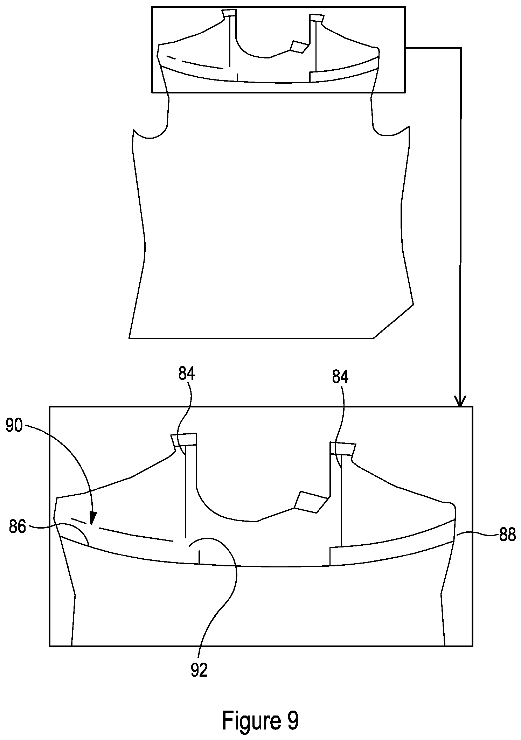

Figure 9 shows a front inside view of an alternative example garment, in the form of a round neck jumper, featuring conductive tracks, which are operable to connect the electrical components of the garment. First conductive tracks 84 adjacent a shoulder seam of the neckline provide electrical connections to the rear of the garment, whilst second and third conductive tracks 86, 88 adjacent respective sleeve seams provide electrical connection to the sleeves of the garment. The second conductive track 86 may, for example, deliver power from energy harvesting yarn within a first sleeve, whilst the third conductive track 88 may deliver power to a stimulation device provided in a second sleeve. A space 90 is provided in the conductive tracks for a textile switch to be built in. Similarly, a further space 92 is provided for a microcontroller to be built in. The conductive tracks are not visible on the exterior of the garment. In the example shown the outside of the garment is plain black so that it is indistinguishable from a regular garment in order to reduce stigma associated with the aesthetic of medical devices when the function is visible.

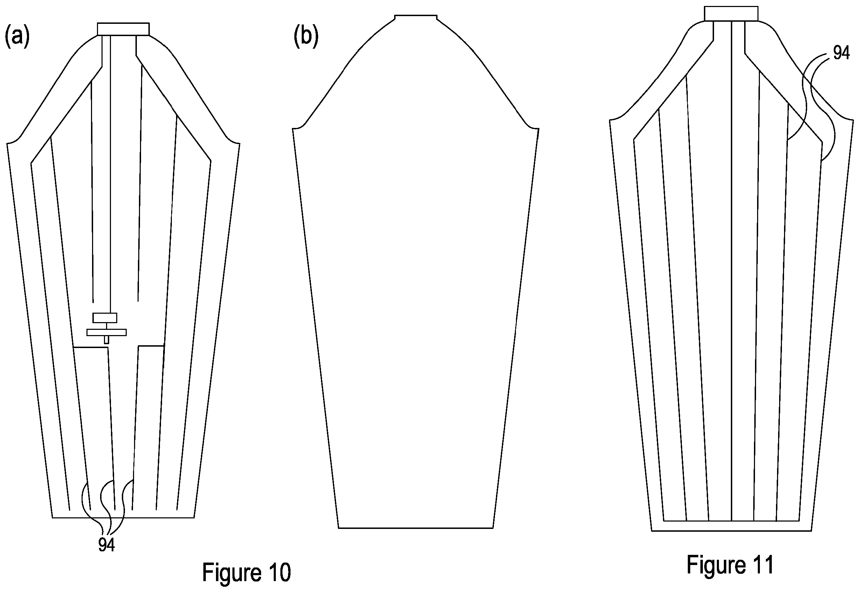

Figure 10 depicts interior and exterior views of a second sleeve. Figure 11 depicts an interior view of a first sleeve. Either sleeve may be a left hand or right hand sleeve, depending on the limb to which it is desired to deliver stimulation. Again, conductive tracks 94 operative to collect energy harvested from the PVDF yarns knit throughout are visible on the interior of both sleeves. Figure 10 further shows conductive tracks operable to connect the microcontroller on the front bodice to the components (EMG sensor and stimulating bead) on the sleeve. In contrast, the sleeve of Figure 11 features conductive tracks to collect energy harvested from the PVDF yarns knit throughout the sleeve, but no connection point for a stimulation device. This sleeve could be worn on the unaffected side of the body and may therefore harvest more energy than the affected side since it is free to move and may move more than usual to compensate for the loss of ability in the affected arm.

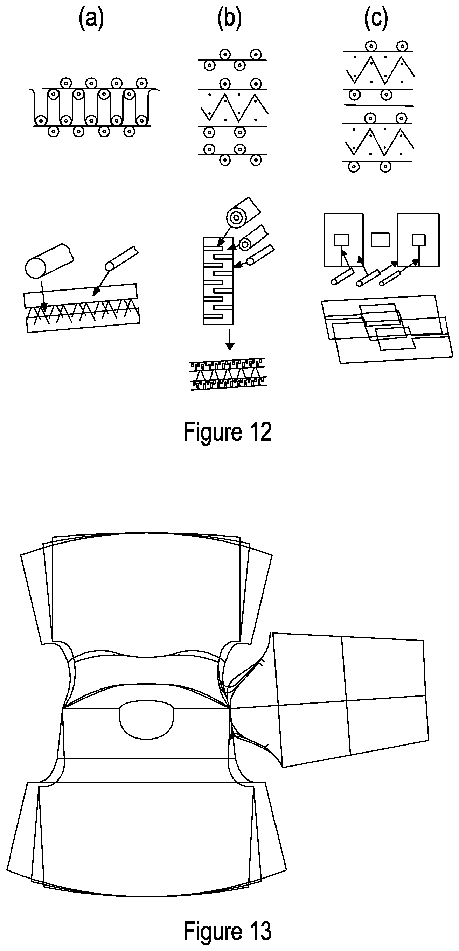

Figure 12 sets out example structures for (a) energy harvesting yarn, (b) a supercapacitor, and (c) an electromyography (EMG) sensor and switch. These are included for context, however it will be appreciated that any suitable capacitor, sensor and/or switch may be used.

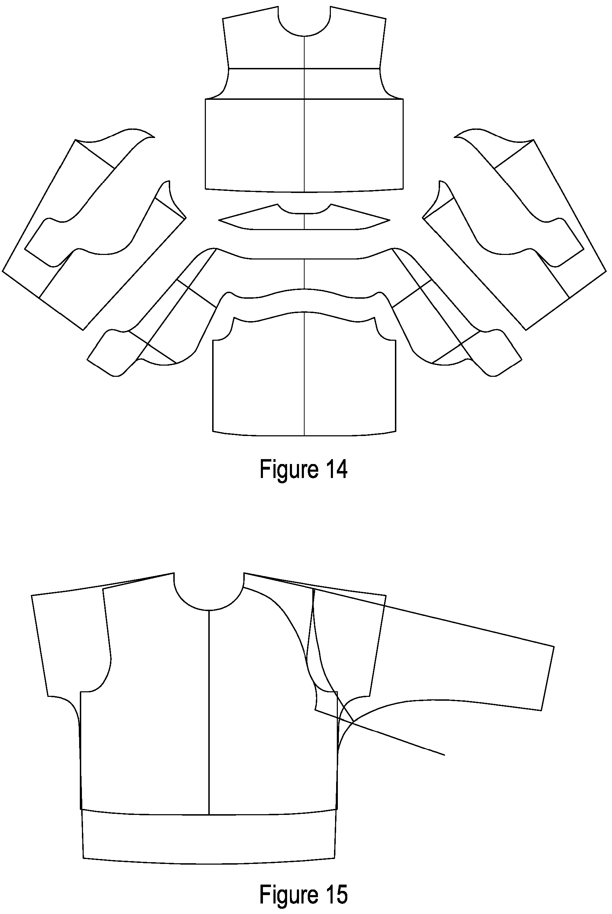

Figure 13 sets out three alternative grown on sleeve head patterns, as well as one more traditional pattern in which the sleeve portions and body portions are separate. Figures 14 and 15 show further pattern variants. It will be appreciated that a large number of pattern variants are available, depending on the requirements for the finished look of the garment. The sleeves need not be connected to the body portions of the garment pattern. However, provision of separate sleeves complicates the garment construction process (as it is necessary to join the electrical connections) and can result in hot spots forming where the conducting yarns are joined at the seams.

In order to ensure the stimulation device is able to deliver stimulation to the selected muscle stimulation site, and that the stimulation delivered sufficient to indent the muscle, the garment is operable to hold the stimulation device closely against the limb of the wearer, such that the stimulation device does not move relative to the stimulation site when the wearer moves their limb. This can be achieved by tensioning the garment, or by varying the combinations of yarns containing, but not limited to elastane or lycra, to influence the stretch ratio of the garment to which the stimulation device is attached to a level sufficient to hold the stimulation device against the wearer's limb through the range of movements that might be expected to be experienced whilst the garment is worn. However, garment tensioning and stretch ratio can increase the amount of force which is needed to push a limb into the garment. This can make dressing difficult, particularly for those suffering from limb weakness.

To alleviate this potential problem, the garment may be constructed such that the yarn tension and/or stretch ratio differs in different regions of the garment. In particular, a region of higher tension and/or stretch ratio may be provided in a band around the selected stimulation device location 52, whilst other portions of the garment, such as the sleeve head, cuff and/or body of the garment may have a lower tension and/or stretch ratio. Regions of differing tension may be created by knitting a garment using a knitting machine in which the tension of the yarn and/or stretch ratio is varied during the knitting process.

Varying the tension and/or stretch ratio in a garment can affect the shaping and overall silhouette of the garment, and particularly a sleeve. In order to maintain a smooth sleeve silhouette different textiles, yarn combinations, textile structures and machine settings may be used for the regions of higher tension to those used for the regions of lower tension. For instance, a higher stretch textile structure and tighter tension (8 for example) may be used to provide a tighter fit, whereas a reduced stretch or a textile structure which has no stretch and a looser tension (12.5 for example) may be used to provide a looser fit.

As discussed above, the bead is preferably required to indent the muscle belly by at least 1mm and therefore a tight fit is required in the region of the stimulation device. However, due to limb weakness and other co-morbidities and movement deficits, a tight garment can be difficult to push the affected limb through the sleeve and therefore make it difficult to dress into. As a result, a varied degree of stretch and tension may be applied to different areas of the sleeve. For example, the tight fit can be assigned to a limited band/ circumference/ area where the bead and EMG sensor is positioned in order to make contact. The fit can be adjusted and loosened working up past the elbow (taking care not to affect the overall silhouette) to limit the area where a greater amount of energy is needed to push the limb through the sleeve.

The tight fit around the bead and sensor is also useful in order to prevent the sleeve from moving around and changing the position of the bead. To support the placement of the sleeve, the sleeve seam (down the arm) provides a guide whereby ensuring that this runs in a straight line down the arm ensures the bead is placed correctly with ease (and through intuitive dressing methods). Shaping of the garment may thus be controlled by using base yarn type and base yarn tension as variables.

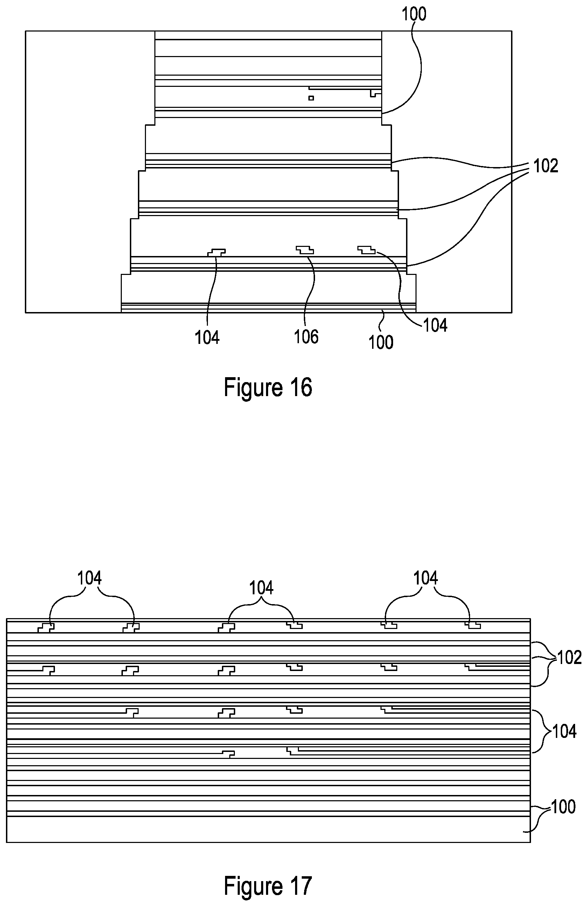

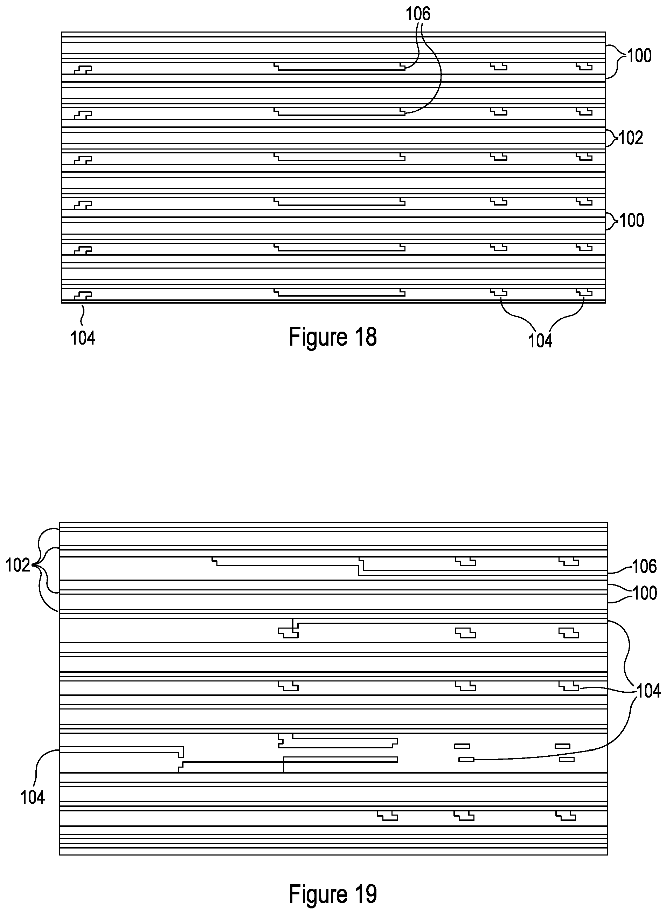

Figures 16-17 illustrate an example sleeve structure at the top of the sleeve (Figure 16) and at the bottom of the sleeve (Figure 17). Figure 18 shows an example sensor structure and Figure 19 shows an example of diverging conductive tracks to direct energy to bead location, just below the EMG sensor. The parameters used to construct the garment portions illustrated in Figures 16-19 are explained in Table 4, below.

**Table 4**

| **Colour in Figure** | **Yarn(s) used** | **textile structure** | **tension at and below bead point/ circumference** | **tension above elbow point** |
|---|---|---|---|---|
| Dark blue (100) | Poly- Zinc (Antibacterial yarn) and/ or lycra | tubular and 1:1 interlock | 10 (tubular) | 11 (tubular) |
| | | | 9 (1:1) | 10 (1:1) |
| Yellow (102) | PVDF piezo yarn | tuck (spacer) | 7 | 7 |
| White, dark grey, green, red, orange and light blue (conductive tracks) (104) | Conductive (100% inox) | single bed | 8 | 8 |
| Pink (EMG sensor) (106) | Conductive (100% inox) | single bed | 9 | 9 |

Again, such a garment could be knitted if required. Alternatively, panels of different textiles could be sewn together to create the areas of different stretch/tension.

A garment 50 including a stimulation device 10 may be worn by a wearer, for example as the wearer goes about their normal life. During movement, the energy harvesting yarns 58 generate electricity from the movement and store that electricity in the capacitor(s) 60.

The EMG sensor(s) 56 monitor electrical activity in a muscle of the wearer and return sensed data to the microcontroller 68. In response to the sensed data the microcontroller 68 may determine that it is necessary to deliver a stimulus to the muscle of the wearer. The sensed data may indicate, for example, that the wearer is able to move a muscle at the stimulation site, or is attempting to move a muscle at the stimulation site.

A muscle tapper may tap only when the muscle becomes active, as detected by electromyogram (EMG). The length of the muscle and rate of change of length of the muscle will be used to deliver rapid taps at micron sensitivities. The EMG recordings may be used to quantify the rate of recovery via the response of the muscle to the stimuli (tap) delivered: EMG recordings are taken from the targeted muscle, from which we can look for short latency responses following the tap stimulus. These should grow with voluntary contraction, and be reduced by vibration of the antagonist muscle, which are the properties expected for a tendon tap reflex.

A further assessment of the long-latency stretch reflex (LLSR) can provide information regarding the impact on the RST. An assessment of the LLSR will be conducted by the sensor to partially measure reticulospinal output since the LLSR appears to have substantial connectivity to the primary motor cortex and the CST.

When it is desired to stimulate the wearer's muscle, the microcontroller activates the stimulation device 10. Electricity is supplied to the electrodes 16, 18 from the capacitor(s) 60, which causes the piezoelectric structure to deform in order to deliver a tap.

Alternatively, the wearer may decide that their muscle requires stimulation. In such a case, the wearer might activate the stimulation device directly using the switch.

In previous work, paired clicks and shocks via transcutaneous electrical stimulation methods have displayed the ability to induce plastic changes in motor pathways. It may be possible to pair taps delivered by the stimulation device described herein with acoustic cues provided via earphones in order to further increase muscle activation. For instance, a sharp tap (e.g. 10ms in duration) may be followed quickly (e.g. 10ms later) by an acoustic cue. This combination may then be repeated again following an interval, e.g. of 1.5s.

Activating the stimulation device continuously to produce a vibration could also elicit a muscle response.

The stimulation device may be driven using some, all or none of the following parameters:
- Range between 0-300Hz with a response speed of 0.002 secs
- Adjusting the frequency range between 1kHz, 3kHz and 6kHz
- A driving voltage in the range of 25-290V
- Limit heat to 50°C limit (to prevent damage to electrodes) with voltage amplifier contained in the microcontroller button

A wearable article of the type described above may permit a user suffering from limb weakness to receive stimulation to that limb as and when needed. Regular muscle stimulation may over time increase their mobility and quality of life, improving long term prognosis.

The stimulation devices described herein may be incorporated into any wearable article, including, but not limited to, upper body garments (such as shirts, blouses, t-shirts, base layers, dresses), lower body garments (such as leggings, tights, trousers, shorts, socks, underwear), bandages/dressings.

The stimulation devices described above include one or more piezoelectric structures and electrodes created using a knitting machine. It will be appreciated that equivalent piezoelectric structures could be created via other methods than the example method described above. For example, a tubular shape could be created from a sheet of mesh that is rolled to create a tube. An advantage of using a continuous circular knitted mesh is that the resulting mesh is continuous, with no breaks or joins. This ensures an even distribution of mechanical force when current is applied to the structure to create a tap. Furthermore, joins can affect conductivity of the nanofibers, which can result in heat spots.

Alternative power sources may be provided to the energy harvesting system described above. For instance, power may be supplied to the microcontroller via a battery. In such a variant the energy harvesting yarn and storage capacitors may be omitted. The power source could be provided inside the stimulation device, for compactness.

It will be appreciated that the controller, capacitors (if present) and switch (if present) need not be located as shown in the attached Figures, but could be located anywhere on the garment that is appropriate in view of the desired finished garment look. If energy harvesting is not used then the controller etc. may be located in the same pattern portion (e.g. sleeve portion) as the stimulation device.

We have appreciated that, in some circumstances, alternative actuators may be used instead of a piezoelectric actuator of the type described above (so long as these actuators may be configured to deliver a tap of sufficient force to deliver a stimulus to a muscle of a wearer, as discussed above).

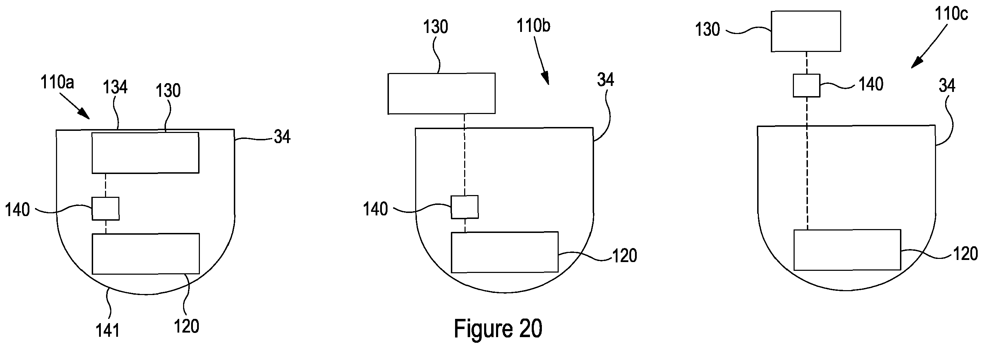

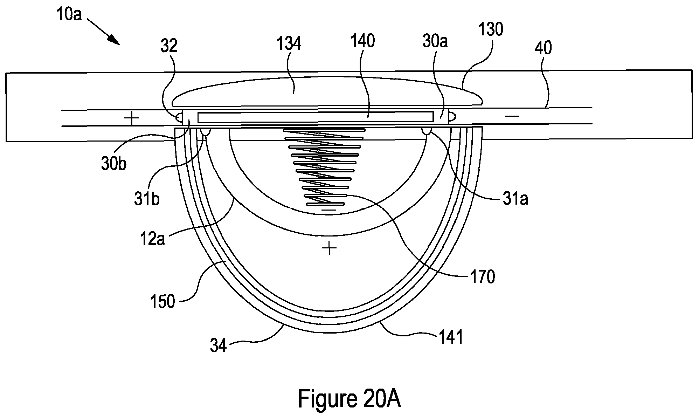

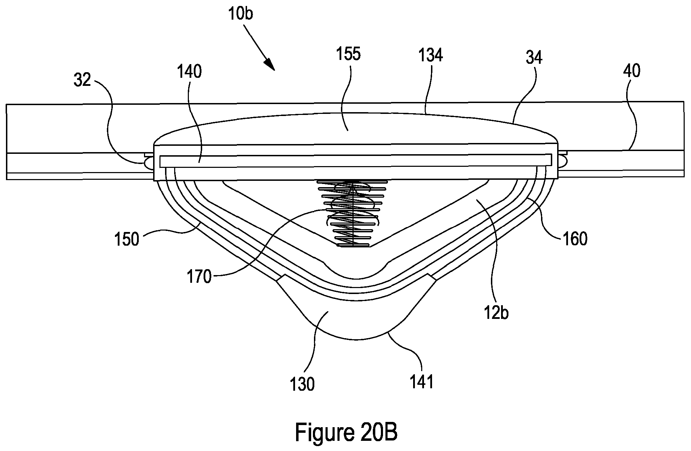

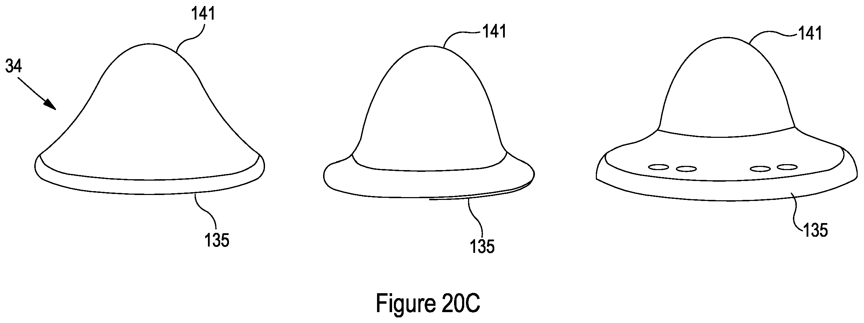

Figure 20 shows examples of stimulation systems 110a, 110b, 110c for a wearable article. Each stimulation system 110a, 110b, 110c comprises an actuator 120 controllable to deliver a mechanical stimulus to a muscle of a wearer of the article, and one or more electromyography sensors 130 operable to sense muscle activity from a wearer.

The actuator 120 may be piezoelectric actuator 12 of the type discussed above. However, as discussed more fully below, other actuators configured to deliver a stimulus to a muscle of a wearer may be used.

The stimulation systems 110c, 110b, 110a each comprise a casing 34. The actuator 12 is enclosed in the casing 34 and is operable to deliver a stimulus to a muscle of the wearer via the casing in the manner discussed above.

The EMG sensor 130 may be enclosed within the casing, or comprised as part of the casing, as shown in system 110a. Alternatively, the EMG sensor may be located outside the casing 34, as in systems 110b and 110c.

The system may include a controller 140, as discussed above. The controller 140 may be enclosed within the casing or comprised as part of the casing, as shown in systems 110a and 110b. Alternatively, the controller may be located outside the casing 34, as in system 110c. Additional EMG sensors may be included in the system if required.

As in the examples discussed above with respect to Figures 1, 1a and 5, the casings 34 of the stimulation devices shown in Figure 20 are shaped to indent into a muscle of a wearer, and thus comprise an indentation region 141 that is shaped to indent a muscle of a wearer by a predefined amount. In each of the stimulation systems shown in Figure 20, the casing 34 has a generally part-ellipsoid shape. That is, rather than being generally spherical or ellipsoid as shown in Figures 1, 1a and 5 above, the casings 34 have a flattened region 134. The flattened region is located on a part of the casing that is opposite to the indentation region 141. The flatted region causes the casing 34, and so the stimulation system, to have a lower profile when attached to a garment.

Alternative stimulation systems 10a, 10b are shown in Figures 20A and 20B. For brevity, like reference numerals are used to refer to like elements in the stimulation device of Figure 1. The stimulation systems 10a, 10b are shown connected to a wearable article 200.

Like the device 10 of Figure 1, the systems 10a, 10b comprise piezoelectric actuators 12a, 12b. In contrast however, the piezoelectric actuators 12a, 12b are part tubular rather than fully tubular. Use of a part tubular actuator allows a portion 134 of a casing 34 of a bead portion of the system to be flattened, as discussed above. In the examples shown in Figures 20A and 20B, the piezoelectric actuators 12a, 12b are part-ellipsoid, and in particular are generally hemispherical.

The piezoelectric actuator 12a in the device of Figure 20A is, like the actuators discussed above, formed of a piezoelectric nanofiber mesh, which may be knitted in a similar manner to that explained above. In contrast, the piezoelectric actuator 12b of Figure 20B is formed of a composite.

The stimulation systems 10a, 10b shown in Figures 20A and 20B each have a casing 34 which, in the specific examples shown, is substantially hemispherical. Such a hemispherical casing comprises a flattened portion 134 and an indentation region 141 which is configured to indent into the flesh of a wearer by a predefined amount (e.g. at least 1mm, at least 2mm, as discussed above).

In each stimulation systems 10a, 10b the part-tubular piezoelectric actuator 12a, 12b is nested within the curved portion of the hemispherical casing 34. Thus, when the actuator deforms in use, the actuator is operable to impart a tap against an inside of the casing adjacent the indentation region (possibly via intermediate plates of the type discussed above).

In a similar manner to the stimulation device 10 discussed above in connection with Figures 1 and 1a, the stimulation systems 10a, 10b of Figures 20A and 20B both comprise a first electrode located on the inside of the piezoelectric actuator and a second electrode located on an outside of the piezoelectric actuator. In the context of a part-tubular actuator, the term "inside" refers to the concave side of the piezo electric actuator and the term "outside" refers to the convex side of the piezoelectric actuator. The electrodes may be knitted of a conductive nanofiber mesh, as discussed above, or may be constructed in another manner (e.g. wire or foil).

Unlike the device shown in Figure 1, both stimulation systems 10a, 10b further comprise an integral EMG sensor 130.

In the case of Figure 20A, the EMG sensor 130 is provided as an electromyography (EMG) sensor layer 130 in the casing 34 of the stimulation device 10a, and in particular in a portion of the casing that is adjacent the flattened region. In the case of Figure 20B, the EMG sensor 130 is embedded within an indentation region 141 of the casing 34. The EMG sensors are thus, in both stimulation devices 10a, 10b, comprised within the casing 34 (albeit in different parts). In each system 10a, 10b, a controller 140 is embedded within a core of the stimulation device 10a, 10b. The controller 140 is connected to the EMG sensor layer 130 via a conductive element 150.

Power may be provided to the actuators 12a, 12b in any suitable manner, such as via energy harvesting yarn (as discussed above), and/or via a battery. In the case of the stimulation system 10b shown in Figure 20B, the casing 34 comprises an energy harvesting portion 155, which may be provided in addition to or instead of an external energy source. The energy harvesting portion 155 of the casing comprises a material, such as PVDF, which is operable to harvest energy from mechanical stimuli. An additional electrode 160 configured to extract charge from the PVDF casing and supply it to the microcontroller elements 140 is provided within the casing. If required, the entire casing may be made from an energy harvesting material, or multiple portions of the casing may be made from such a material. The casing may be 3D printed.

Both stimulation systems 10a, 10b may further include a spring 170. The spring 170 abuts the piezoelectric actuator 12a, 12n so that, when the piezoelectric actuator 12 mechanically deforms, it compresses the spring 2. The spring 2 stores the energy from the piezoelectric actuator 12 and releases it suddenly to deliver a force to the casing 34.

Figure 20C illustrates three further variants of casings 34 having a flattened region. In these examples the flattened region extends to a diameter that is wider than the diameter of a widest part of the indentation region (which is, in the examples shown, ellipsoid). The examples shown thus have a flared flattened region 135, giving the casing a generally bell-shaped cross-section. The purpose of the flared flattened region is to provide additional stability to the casing when used as a bead, by preventing it from rotating onto its side when in use and thus maintaining the tap into the limb in a perpendicular direction. The flared region an also provide a larger area in which to accommodate thread holes.

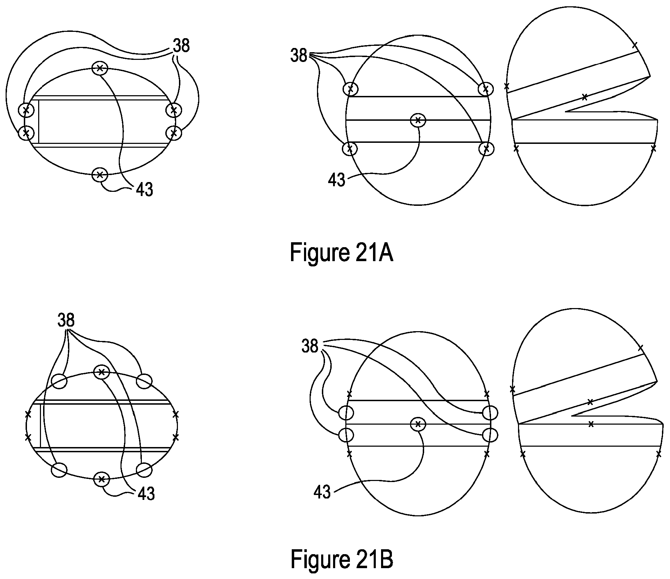

Figures 21A and 21B show alternative embodiments for casings 34a, 34b. The leftmost diagrams show top-down views of the casings 34a, 34b. The central and rightmost diagrams show side views of the casings 34a, 34b, where the rightmost diagram shows the casings opened. The casings 34a, 34b are formed in two parts, and are configured to open and close so that they may be connected to a garment. For example, the two parts of the casing may be hinged together so as to clip or snap closed around a yarn. The two parts may connect together in any suitable manner, e.g. via a press fit, screw fit or using an adhesive.

The casings 34a, 34b include thread holes 38 for connecting the casing to a yarn of a garment. The thread holes 38 may be defined at a seam of the casing, so that a recess on each part of the casing cooperates to form a thread hole when the two parts are joined. In this manner the casing may clip around a yarn and move freely on the yarn once closed. Alternatively, thread holes may be provided in one part or the other of the casing, as discussed in the examples above.

The casings 34a and 34b further include centre holes 43. The centre holes may permit a conductive yarn 28 to pass into and/or out of the casing, e.g. for electrical connection.

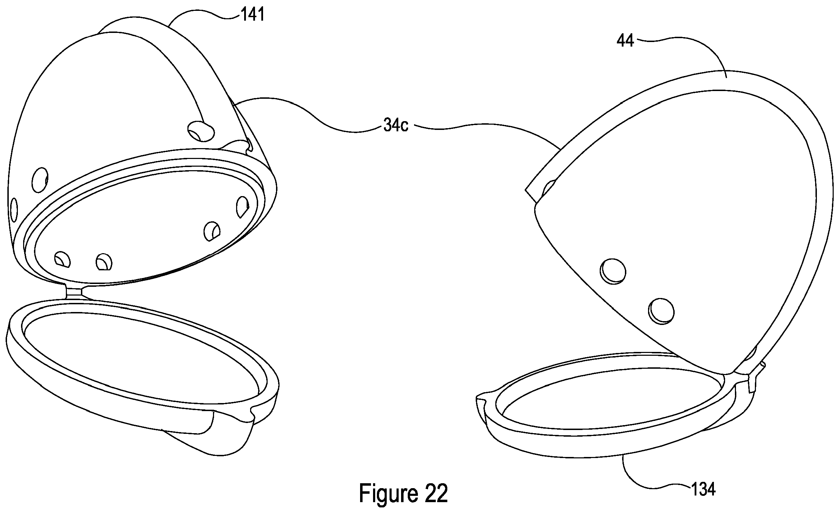

Figure 22 shows a further example of a casing 34 which can be used for the stimulation device 10. Like the casings of Figures 20, 20A and 20B the casing 34c is part-ellipsoid and includes a flatted region having thread holes adjacent the flattened region. Like the casings 34a and 34b of Figures 21A and 21B the casing 34c is comprised of two hinged parts, which are configured to clip closed via a press fit. The thread holes 38 are located in a curved part of the casing rather than on a seam between the two parts. The casing 34 comprises a raised rib 44 in the indentation region 141.

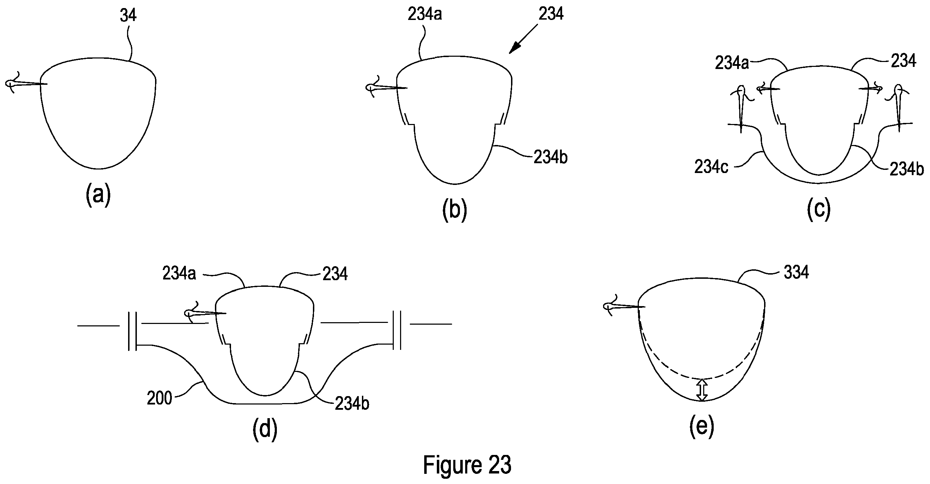

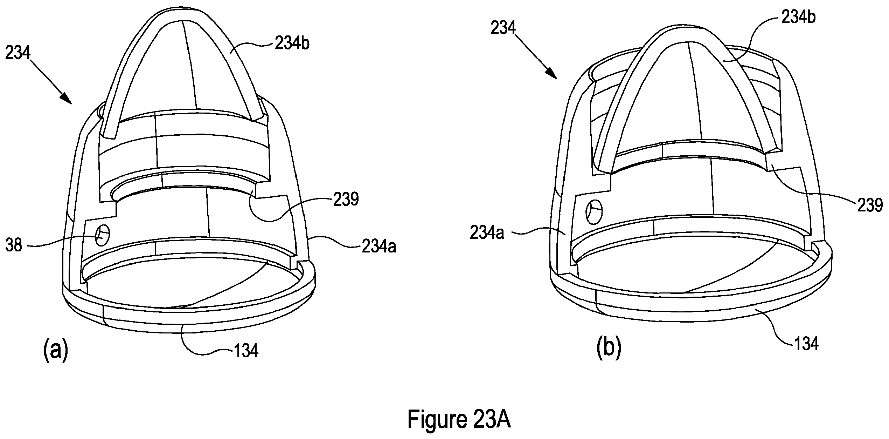

Figure 23 shows five alternative casing 34 designs for a stimulation device 10.

Design (a) in Figure 23 shows a simple hemi-spherical casing 34. The casing 34 has either a hard surface or a soft surface for the actuator to tap against. In this embodiment, the casing is unitary. An indentation region of the casing 34 is shaped to transfer force to a user when the actuator taps against interior of the casing 34.

Design (b) in Figure 23 shows a hemispherical casing 234 formed of two parts, 234a and 234b which are moveable relative to each other. A tip section 234b is captive within a base section 234a and configured to move into and out of the base section. In use, the base section 34a may be secured to a garment. An actuator within the casing is able to deliver a tap to the user via the moveable tip section 234b. Permitting relative movement between casing components in this manner can permit a more compact system to be provided for a given force of tap.

Design (c) in Figure 23 is similar to design (b), but an additional flat, rigid surface 234c is located beneath the casing 234. The casing 234 can hit or tap against the flat rigid surface, which in turns delivers a force to the user.

Design (d) in Figure 23 is similar to design (c), but the flat, rigid surface is replaced with the textile 200 the garment is made from. That is, the casing 234 may be located between two layers of a multilayer garment. The yarn composition may be configured to stiffen the material of at least the lower garment layer (through which the tap is delivered) to help deliver the force or tap to the user.

Design (e) in Figure 23 shows a casing 334 which is deformable so that a tip of the casing deforms when it is subject to a tap or force from the actuator. The casing 34 may be formed from or comprise elastane, or any other suitable material that is both flexible enough to deform but hard enough not to absorb the force of the tap.

Figure 23A shows more detail of the two-part casing 234 having relative movement between a tip section 234b and a base section 234a, similar to those shown in Figure 23 designs (b), (c) and (d). Figure 23A shows a cross section through an exemplary two part casing, in an indentation position (picture (a)) and a retracted position (picture (b)). The mechanical deformation of the bead tip when pushed outward by an actuator (not shown) is clearly demonstrated in picture (a) in contrast to the retracted position when undeformed by an internal actuator, the tip portion 234b instead being pushed inwards by contact with a muscle of a wearer. The casing further includes an internal stop feature 236, which restricts the extent of the inward movement of the tip section 234b, so as to avoid damage to the internal components of the bead. The base section may have a hinged construction of the type discussed above with respect to Figure 22, or may be formed as a single piece.

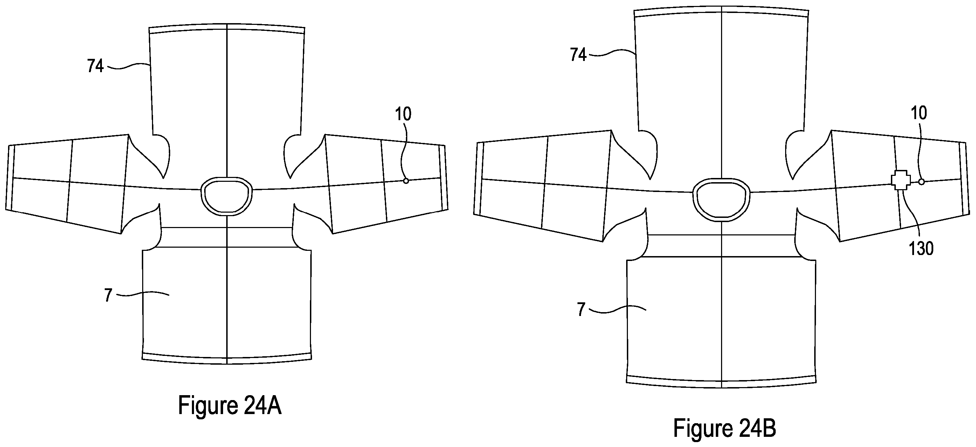

Figures 24a and 24b show alternative garment patterns 74 to those depicted in Figures 8, 13, 14 and 15. In Figure 24a, the pattern 74 is simplified greatly compared with the pattern 74 of Figure 8 because the EMG and controller components are contained within the stimulation device 10. In Figure 24b, the pattern 74 is similar to that in Figure 24a, but the EMG sensor 3 is external from the stimulation device 10. The features of each pattern are interchangeable with the patterns discussed above, for example the controller could be located elsewhere on the garment in the patterns shown in Figures 24a and 24b. Further, one or more energy harvesting wires could be included if required.

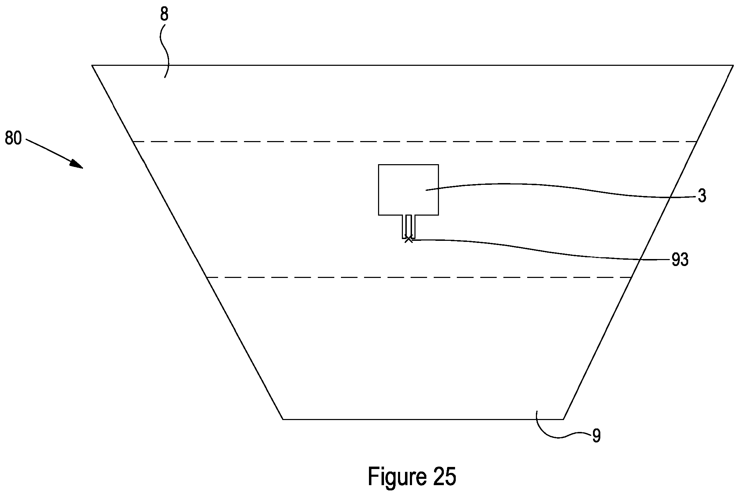

Figure 25 shows a section of a sleeve garment structure 80 in accordance with an embodiment of the invention. The structure comprises a top section 8 and a bottom section 9, where the top section 8 approaches the body of the garment and the bottom section approaches the cuff of the sleeve. The top 8 section and bottom section 9 have lower tension and differing stretch ratio than a central section of the sleeve structure. The structure is designed so that the central section has a tighter fit on the user than the top section 8 and bottom section 9, as discussed in detail above. The tight fit of the central section keeps the EMG sensor 3 and bead in close contact with the user. First and second conductive tracks of the EMG sensor are connected via a float switch 93.

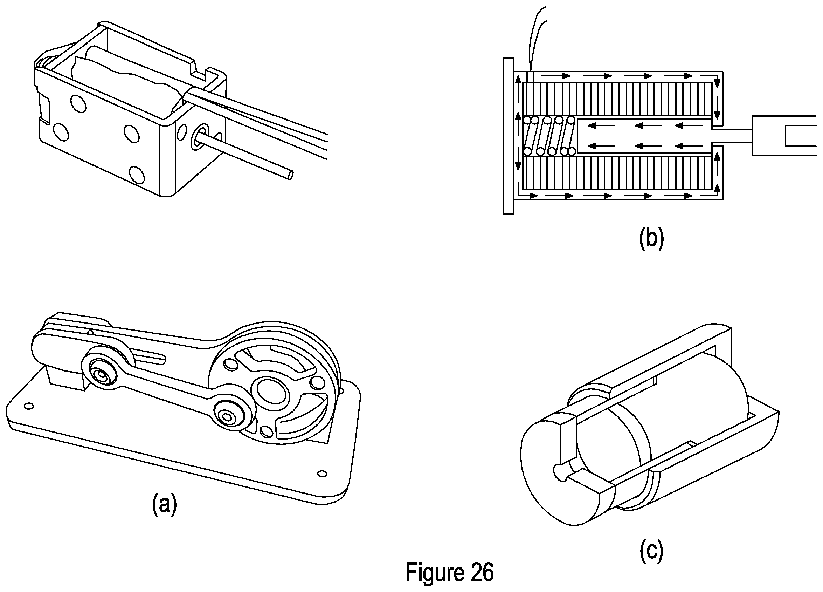

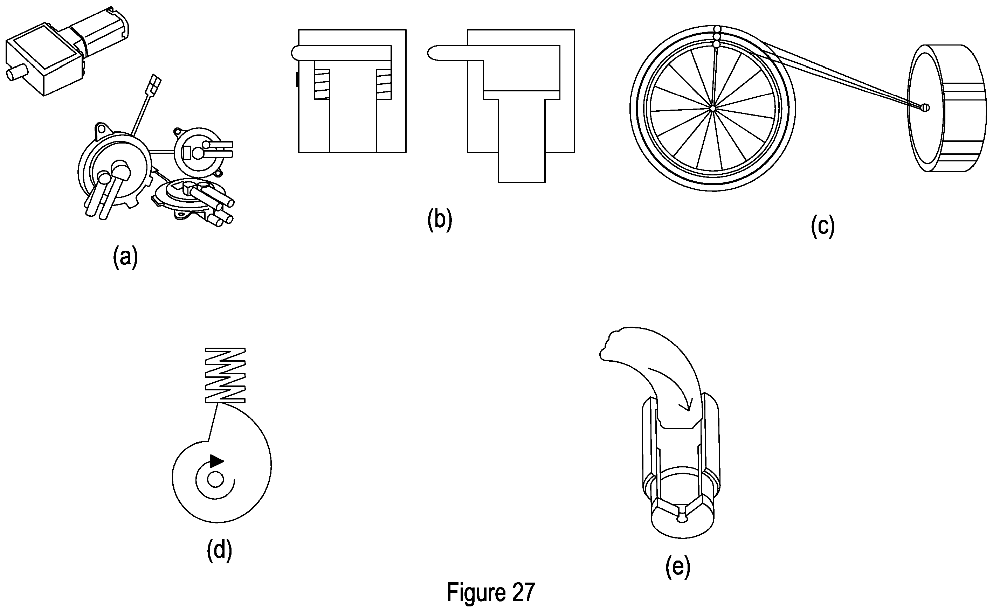

As noted above, a stimulation system of the type described herein may still be configured to provide beneficial muscle stimulation even without a piezoelectric actuator - other actuators may be used instead. Figures 26 and 27 show examples of such alternative actuators.

For example, an example of an electronic actuator is shown in part (a) of Figure 26. The electronic actuator could be driven using a solenoid or a motor. Solenoid powered actuators may provide higher initial forces than other actuators but over a shorter distance such as required between system held in the garment adjacent to the arm and the muscle beneath the skin. Voice coil powered actuators may provide a lower peak force but maintain a constant force over the entire stroke (throughout the actuation). A solenoid actuator is shown in part (b) of Figure 26. A voice coil actuator is shown in part (c) of Figure 26. Alternatively, a fluidic actuator may be used. Such an actuator may be driven by the movement of fluids. The actuator may be powered by a pressure reservoir. The actuator may be powered by a pump. Multiple fluidic actuators may be used, and they may all be powered using a single pump or pressure reservoir. An example of a fluidic actuator is shown in part (a) of Figure 27.

Pneumatic actuators may be used. Pneumatic actuators typically use a pressurised gas to drive a piston, or inflate a cavity. When operating at low actuation frequency, a single pressurised gas cartridge, such as a CO2 cartridge, may allow for continuous actuation for several hours. An example of a pneumatic actuator is shown in part (b) of Figure 27.

Crank arm actuators may be used. In such actuators, a rotating wheel may rotate to drive a piston to deliver an impulse. An example crank arm actuator is shown in part (c) of Figure 27.

Spring release actuators may be used. In such actuators, a motor may compress a spring which is configured to deliver an impulse via a release mechanism when required. An example spring release actuator is shown in part (d) of Figure 27.

Curved actuators may be used. The piston being actuated by the actuator may be curved to reduce the overall size of the stimulation device. Having curved actuators may allow the stimulation device to lie flat against the body. A plurality of curved actuators may be used to compensate for the reduced force achieved from curving an actuator. The plurality of curved actuators may be operated simultaneously to provide a greater cumulative resultant force. An example curved actuator is shown in part (e) of Figure 27.

According to the invention any actuator of the described system should be able to deliver a force of at least 1 Newton over a displacement of at least 1 mm in order to deliver a tap sufficient to stimulate a muscle of a wearer.

The table below shows example actuator criteria:

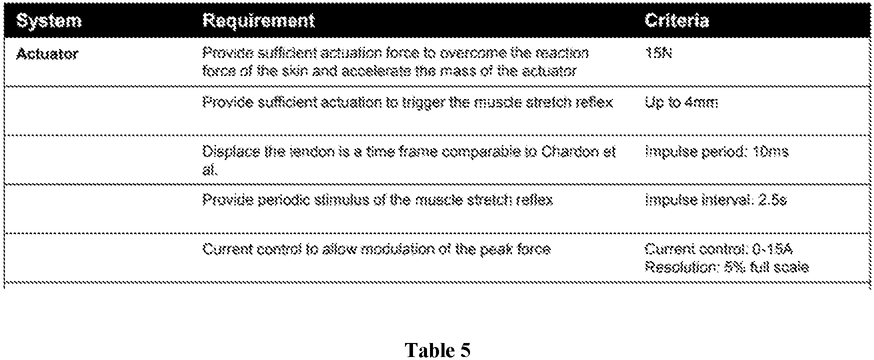

The casings discussed above have been primarily ellipsoid or part ellipsoid. These shapes have been chosen so as to provide good indentation into a muscle of a wearer whilst simultaneously maximising the force transferred to the casing by an internal tubular or part tubular actuator. It will be appreciated however that the casing could have other shapes if required, particularly if housing a non-tubular actuator. In the case of a tubular or part -tubular actuator, the casing may be internally tubular or part-tubular, but may have a different exterior shape.

Such an external shape should comprise an indentation region that is operable to indent into a user's muscle by a pre-specified amount, such as 1mm, 2mm, 3mm, 4mm, 5mm or more. This means that any garment connections, such as thread holes, should be located at least 1mm, 2mm, 3mm, 4mm, 5mm or more from a tip of the casing, to ensure that the indentation region of the casing can be pushed into the muscle of the user when incorporated into a garment worn by the user.

Preferably any such casing shape should avoid sharp edges in the indentation region.

## Claims

1. A stimulation system (10) for a wearable article (50), the stimulation system comprising:
an actuator (12) controllable to deliver a mechanical stimulus to a muscle of a wearer of the article;
one or more electrodiagnostic sensors (56) operable to sense muscle activity from a wearer; and
a casing (34) enclosing the actuator;
wherein the actuator is configured to provide a tap with a force of at least 1 Newton over a displacement of at least 1 mm so as to cause an indentation in the wearer's flesh.

2. The stimulation system of claim 1, wherein one or more of the electrodiagnostic sensors are electromyography sensors.

3. The stimulation system of claim 1 or claim 2, wherein the actuator is a piezoelectric actuator (14).

4. The stimulation system of claim 3, wherein the actuator comprises a structure formed of piezoelectric nanofiber mesh, wherein the structure is tubular;, and wherein the piezoelectric actuator further comprises a first electrode (16) and a second electrode (18), wherein the first electrode is located on an inside of the tubular structure and the second electrode is located on an outside of the tubular structure; and, optionally or preferably,
wherein the first electrode comprises a first electrode structure formed of conductive nanofiber mesh, and the second electrode comprises a second electrode structure formed of conductive nanofiber mesh, the first electrode structure being located at least partially inside the second electrode structure and offset from the second electrode structure.

5. The stimulation system of any preceding claim, wherein the casing encloses or comprises the one or more electrodiagnostic sensors.

6. The stimulation system of claim 5, wherein the casing comprises an indentation region (41) operable to indent a muscle of a wearer, and wherein the actuator is operable to tap against an interior of the casing at least at the indentation region.

7. The stimulation system of claim 4, or any claim dependent on claim 4, wherein the stimulation system comprises one or more electrical connectors operable to connect the first and second electrodes to a power source.

8. The stimulation system of any of claims 5-7, wherein the casing has a moveable part (234a, b) configured to increase the strength of the mechanical stimulus provided to the user.

9. A wearable article comprising the stimulation system of any preceding claim.

10. The wearable article of claim 9, wherein the article comprises a power source comprising one or more energy harvesting yarns and at least one capacitor operable to store electrical energy harvested by the one or more energy harvesting yarns, and, optionally or preferably, wherein the article comprises two layers, and the energy harvesting yarns are located between the two layers.

11. The wearable article of claim 9 or claim 10, further comprising a controller (140) operable to control the stimulation system to deliver the mechanical stimulus to the muscle of the wearer.

12. The wearable article of any of claims 9-11wherein the wearable article is shaped to be worn adjacent a selected muscle stimulation site, and the stimulation system is located at a selected location on the wearable article so as to deliver mechanical stimulation to the selected muscle stimulation site when the wearable garment is worn by the wearer.

13. The wearable article of any of claims 9-12, wherein the wearable article is a garment.

14. The wearable article of claim 13, wherein the stimulation system is located at a selected location on a sleeve of the garment so as to deliver mechanical stimulation to a selected muscle stimulation site in an upper limb of the wearer.

15. The wearable article of claim 14, wherein the wearable article includes a first region of having a first tension and a second region having a second tension, wherein the selected location is provided in the first region and the first tension is higher than the second tension.

## Patentansprüche

1. Stimulationssystem (10) für einen tragbaren Artikel (50), wobei das Stimulationssystem Folgendes umfasst:
einen Aktor (12), der dazu steuerbar ist, einen mechanischen Reiz an einen Muskel eines Trägers des Artikels abzugeben;
einen oder mehrere elektrodiagnostische Sensoren (56), die dazu betreibbar sind, die Muskelaktivität eines Trägers zu erfassen; und
ein Gehäuse (34), das den Aktor umschließt;
wobei der Aktor dazu konfiguriert ist, einen Klopfimpuls mit einer Kraft von mindestens 1 Newton über einen Weg von mindestens 1 mm bereitzustellen, um eine Eindrückung in die Haut des Trägers zu bewirken.

2. Stimulationssystem nach Anspruch 1, wobei einer oder mehrere der elektrodiagnostischen Sensoren Elektromyographie-Sensoren sind.

3. Stimulationssystem nach Anspruch 1 oder Anspruch 2, wobei der Aktor ein piezoelektrischer Aktor (14) ist.

4. Stimulationssystem nach Anspruch 3, wobei der Aktor eine Struktur umfasst, die aus piezoelektrischem Nanofasernetz gebildet ist, wobei die Struktur röhrenförmig ist;, und wobei der piezoelektrische Aktor weiter eine erste Elektrode (16) und eine zweite Elektrode (18) umfasst, wobei die erste Elektrode sich an einer Innenseite der röhrenförmigen Struktur befindet und die zweite Elektrode sich an einer Außenseite der röhrenförmigen Struktur befindet; und optional oder bevorzugt,
wobei die erste Elektrode eine erste Elektrodenstruktur umfasst, die aus einem leitfähigen Nanofasernetz gebildet ist, und die zweite Elektrode eine zweite Elektrodenstruktur umfasst, die aus einem leitfähigen Nanofasernetz gebildet ist, wobei sich die erste Elektrodenstruktur zumindest teilweise innerhalb der zweiten Elektrodenstruktur befindet und gegenüber der zweiten Elektrodenstruktur versetzt ist.

5. Stimulationssystem nach einem der vorstehenden Ansprüche, wobei das Gehäuse den oder die elektrodiagnostischen Sensoren umschließt oder umfasst.

6. Stimulationssystem nach Anspruch 5, wobei das Gehäuse einen Eindruckbereich (41) umfasst, der dazu betreibbar ist, einen Muskel eines Trägers einzudrücken, und wobei der Aktor betreibbar ist, um zumindest im Eindruckbereich gegen ein Innere des Gehäuses zu klopfen.

7. Stimulationssystem nach Anspruch 4 oder jeglichem Anspruch, der von Anspruch 4 abhängt, wobei das Stimulationssystem eine oder mehrere elektrische Verbindungselemente umfasst, die dazu betreibbar sind, die erste und zweite Elektrode mit einer Stromquelle zu verbinden.

8. Stimulationssystem nach einem der Ansprüche 5-7, wobei das Gehäuse einen beweglichen Teil (234a, b) aufweist, der dazu konfiguriert ist, die Stärke des an den Benutzer bereitgestellten mechanischen Reizes zu erhöhen.

9. Tragbarer Artikel, umfassend das Stimulationssystem nach einem der vorstehenden Ansprüche.

10. Tragbarer Artikel nach Anspruch 9, wobei der Artikel eine Stromquelle umfasst, die eine oder mehrere Energiegewinnungsgarne und mindestens einen Kondensator umfasst, der dazu betreibbar ist, von der einen oder den mehreren Energiegewinnungsgarnen gewonnene elektrische Energie zu speichern; und optional oder bevorzugt, wobei der Artikel zwei Schichten umfasst, und sich die Energiegewinnungsgarne zwischen den beiden Schichten befindet.

11. Tragbarer Artikel nach Anspruch 9 oder Anspruch 10, weiter umfassend eine Steuereinheit (140), die dazu betreibbar ist, das Stimulationssystem zu steuern, um den mechanischen Reiz an den Muskel des Trägers abzugeben.

12. Tragbarer Artikel nach einem der Ansprüche 9-11, wobei der tragbare Artikel so geformt ist, dass er an einer ausgewählten Muskelstimulationsstelle getragen werden kann, und das Stimulationssystem an einer ausgewählten Stelle auf dem tragbaren Artikel angeordnet ist, um eine mechanische Stimulation an die ausgewählte Muskelstimulationsstelle abzugeben, wenn das tragbare Kleidungsstück vom Träger getragen wird.

13. Tragbarer Artikel nach einem der Ansprüche 9-12, wobei der tragbare Artikel ein Kleidungsstück ist.

14. Tragbarer Artikel nach Anspruch 13, wobei sich das Stimulationssystem an einer ausgewählten Stelle an einem Ärmel des Kleidungsstücks befindet, um eine mechanische Stimulation an eine ausgewählte Muskelstimulationsstelle in einem oberen Gliedmaß des Trägers abzugeben.

15. Tragbarer Artikel nach Anspruch 14, wobei der tragbare Artikel einen ersten Bereich, der eine erste Spannung aufweist, und einen zweiten Bereich, der eine zweite Spannung aufweist, einschließt, wobei die ausgewählte Stelle in dem ersten Bereich bereitgestellt ist und die erste Spannung höher ist als die zweite Spannung.

## Revendications

1. Système (10) de stimulation pour un article pouvant être porté (50), le système de stimulation comprenant :
un actionneur (12) pouvant être commandé pour administrer un stimulus mécanique à un muscle d'un porteur de l'article ;
un ou plusieurs capteurs (56) d'électrodiagnostic pouvant être utilisés pour détecter l'activité musculaire d'un porteur ; et
un boîtier (34) enfermant l'actionneur ;
dans lequel l'actionneur est configuré pour donner une tape avec une force d'au moins 1 Newton sur un déplacement d'au moins 1 mm afin de provoquer un enfoncement dans la chair du porteur.

2. Système de stimulation selon la revendication 1, dans lequel un ou plusieurs des capteurs d'électrodiagnostic sont des capteurs d'électromyographie.

3. Système de stimulation selon la revendication 1 ou la revendication 2, dans lequel l'actionneur est un actionneur piézoélectrique (14).

4. Système de stimulation selon la revendication 3, dans lequel l'actionneur comprend une structure formée d'un maillage de nanofibre piézoélectrique, dans lequel la structure est tubulaire ; et dans lequel l'actionneur piézoélectrique comprend en outre une première électrode (16) et une seconde électrode (18), dans lequel la première électrode est située sur un intérieur de la structure tubulaire et la seconde électrode est située sur un extérieur de la structure tubulaire ; et, optionnellement ou de préférence,
dans lequel la première électrode comprend une première structure d'électrode formée de maillage de nanofibre conductrice, et la seconde électrode comprend une seconde structure d'électrode formée de maillage de nanofibre conductrice, la première structure d'électrode étant située au moins partiellement à l'intérieur de la seconde structure d'électrode et décalée par rapport à la seconde structure d'électrode.

5. Système selon une quelconque revendication précédente, dans lequel le boîtier enferme ou comprend les un ou plusieurs capteurs d'électrodiagnostic.

6. Système de stimulation selon la revendication 5, dans lequel le boîtier comprend une région (41) d'enfoncement pouvant être utilisée pour enfoncer un muscle d'un porteur, et dans lequel l'actionneur est être utilisé pour taper contre un intérieur du boîtier au moins au niveau de la région d'enfoncement.

7. Système de stimulation selon la revendication 4, ou une quelconque revendication dépendant de la revendication 4, dans lequel le système de stimulation comprend un ou plusieurs connecteurs électriques pouvant être utilisés pour connecter les première et seconde électrodes à une source d'alimentation.

8. Système de stimulation selon l'une quelconque des revendications 5 à 7, dans lequel le boîtier présente une partie mobile (234a, b) conçue pour augmenter la force du stimulus mécanique fourni à l'utilisateur.

9. Article pouvant être porté comprenant le système de stimulation selon une quelconque revendication précédente.

10. Article pouvant être porté selon la revendication 9, dans lequel l'article comprend une source d'alimentation comprenant un ou plusieurs fils de récupération d'énergie et au moins un condensateur pouvant être utilisé pour stocker l'énergie électrique récupérée par les un ou plusieurs fils de récupération d'énergie ; et, optionnellement ou de préférence, dans lequel l'article comprend deux couches, et les fils de récupération d'énergie sont situés entre les deux couches.

11. Article pouvant être porté selon la revendication 9 ou la revendication 10, comprenant en outre un dispositif (140) de commande pouvant être utilisé pour commander le système de stimulation pour administrer le stimulus mécanique au muscle du porteur.

12. Article pouvant être porté selon l'une quelconque des revendications 9 à 11,dans lequel l'article pouvant être porté est conçu pour être porté adjacent à un site de stimulation musculaire sélectionné, et le système de stimulation est situé au niveau d'un emplacement sélectionné sur l'article pouvant être porté afin d'administrer une stimulation mécanique au site de stimulation musculaire sélectionné lorsque le vêtement pouvant être porté est porté par le porteur.

13. Article pouvant être porté selon l'une quelconque des revendications 9 à 12, dans lequel l'article pouvant être porté est un vêtement.

14. Article pouvant être porté selon la revendication 13, dans lequel le système de stimulation est situé au niveau d'un endroit sélectionné sur une manche du vêtement afin d'administrer une stimulation mécanique à un site de stimulation musculaire sélectionné dans un membre supérieur du porteur.

15. Article pouvant être porté selon la revendication 14, dans lequel l'article pouvant être porté inclut une première région présentant une première tension et une seconde région présentant une seconde tension, dans lequel l'emplacement sélectionné est disposé dans la première région et la première tension est supérieure à la seconde tension.
